(19) Europäisches Patentamt
European Patent Office
Office européen des brevets

(11) EP 2 184 073 A1

(12) **EUROPEAN PATENT APPLICATION**
published in accordance with Art. 153(4) EPC

(43) Date of publication:
**12.05.2010 Bulletin 2010/19**

(21) Application number: **08792608.5**

(22) Date of filing: **21.08.2008**

(51) Int Cl.:
***A61K 45/00*** *(2006.01)* ***A61K 31/047*** *(2006.01)*
***A61K 31/12*** *(2006.01)* ***A61K 31/165*** *(2006.01)*
***A61K 31/216*** *(2006.01)* ***A61K 31/4402*** *(2006.01)*
***A61K 31/4406*** *(2006.01)* ***A61K 31/445*** *(2006.01)*
***A61K 31/472*** *(2006.01)* ***A61K 31/495*** *(2006.01)*
***A61K 31/5375*** *(2006.01)* ***A61P 35/00*** *(2006.01)*
***A61P 35/02*** *(2006.01)* ***A61P 43/00*** *(2006.01)*
***C07D 213/30*** *(2006.01)* ***C07D 213/40*** *(2006.01)*
***C07D 217/06*** *(2006.01)* ***C07D 295/08*** *(2006.01)*
***C07D 295/16*** *(2006.01)*

(86) International application number:
**PCT/JP2008/064887**

(87) International publication number:
**WO 2009/028387 (05.03.2009 Gazette 2009/10)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **24.08.2007 JP 2007217880**

(71) Applicant: **Kyowa Hakko Kirin Co., Ltd.**
**Tokyo 100-8185 (JP)**

(72) Inventors:
- **NAKASHIMA, Takayuki**
  **Sunto-gun, Shizuoka 411-8731 (JP)**
- **SHIOTSU, Yukimasa**
  **Sunto-gun, Shizuoka 411-8731 (JP)**

(74) Representative: **Dossmann, Gérard et al**
**Casalonga & Partners**
**Bayerstrasse 71-73**
**80335 München (DE)**

(54) **THERAPEUTIC AGENT FOR CANCER WITH RESISTANCE TO PROTEASE INHIBITOR**

(57) The present invention provides therapeutic agents for a cancer resistant to a protease inhibitor which comprises, as an active ingredient, a benzoyl compound represented by formula (I):

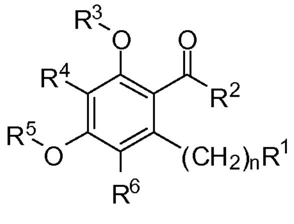

(I)

[wherein n represents an integer of 1 to 5;
$R^1$ represents substituted or unsubstituted lower alkyl, $CONR^7R^8$ (wherein $R^7$ and $R^8$, which may be the same or different, each represent a hydrogen atom, substituted or unsubstituted lower alkyl, or the like), or the like;
$R^2$ represents substituted or unsubstituted aryl or the like;
$R^3$ and $R^5$, which may be the same or different, each represent a hydrogen atom, substituted or unsubstituted lower alkyl, or the like;
$R^4$ represents a hydrogen atom or the like; and
$R^6$ represents a hydrogen atom, halogen, substituted or unsubstituted lower alkyl, or the like] or a pharmaceutically acceptable salt thereof as a heat shock protein 90 (Hsp90) family protein inhibitor, and the like.

EP 2 184 073 A1

## Description

### Technical Field

The present invention relates to a therapeutic agent for a cancer resistant to a protease inhibitor which comprises, as an active ingredient, a heat shock protein 90 (hereinafter Hsp90) family protein inhibitor, and the like.

### Background Art

Hsp is a series of proteins expressed in cells when the cells are exposed to a stress environment such as heat shock, and are classified into families such as Hsp90, Hsp70 and Hsp60 according to their molecular weight. These proteins are also called molecular chaperones, and generally, folding, membrane penetration, association, aggregation suppression of proteins, etc. are recognized as their main functions.

Hsp90 is a general term for Hsps having a molecular weight of about 90kDa. As the Hsp90 family of eukaryotes, Hsp90α, Hsp90β, Grp94, Hsp75/TRAP1. etc., have been identified. These Hsps belonging to the Hsp90 family are hereinafter generically called Hsp90.

In recent years, it has been clarified that Hsp90 forms a complex specifically with a molecule involved in cell growth and tumorigenesis to participate in the cell cycle, cell growth, cell survival, cell immortalization, angiogenesis, metastasis and invasion. Proteins forming a complex specifically with Hsp90 are called Hsp90 client proteins. In order to maintain the function and stability of the Hsp90 client proteins in the cell, interaction with Hsp90 is considered to be necessary.

As a molecular mechanism associated with development and malignant conversion of a variety of cancers and the mechanism of drug resistance have been elucidated in recent years, the studies of molecular target therapies related to treatment of cancers have actively been performed by directly controlling the particular function of proteins expressed in cancer cells and tumor environment. Some of the molecular-targeted drugs have already been applied clinically and attracted a great deal of attention to a high effectiveness and a relatively weak adverse reaction in comparison with those of existing chemotherapeutic agents. It has been elucidated, however, that the molecular-targeted drugs also cause to develop resistant cancers similarly to the chemotherapeutic agents [Current Medicinal Chemistry, 14, 223-232 (2007)]. In practice, for important molecular targets, receptor-type tyrosine kinases (EGF receptor, ErbB-2, KIT, etc.) and fusion proteins (Bcr-Abl, etc.) derived from gene translocation, there is a problem that the generation of resistance for these molecular targeted drugs has been observed in clinical human tumors. On the other hand, the above-mentioned receptor-type tyrosine kinases and fusion proteins are client proteins for Hsp90, and thus, Hsp90 inhibitors may induce decomposition of these receptor-type tyrosine kinases and fusion proteins to inhibit their function.

Chronic myeloid leukemia is a leukemia caused by chromosomal translocation t (9;22) termed Philadelphia chromosome. Constant activation of Bcr-Abl formed by this gene translocation has been considered as the cause of chronic myeloid leukemia since it shows excessive growth and anti-apoptosis actions. Imatinib (Glivec) is an inhibitor for Bcr-Abl, which shows a dramatic effect for chronic myeloid leukemia patients irresponsive to interferon and has been applied clinically [New England Journal of Medicine, 344, 1084-1086 (2001); New England Journal of Medicine, 344, 1031-1037 (2001)]. Although the effect lasts in patients at the chronic stage, the effect for blast crisis patients is transient and in both cases, a resistant cancer gradually develops. As for the cause of resistance development, a lowering of sensitivity to imatinib due to amplification of the Bcr-Abl gene and mutation of the kinase domain (point mutation of T315I, E255K, etc.) is reported [Blood, 99, 3472-3475 (2002) ; Leukemia, 17, 829-838 (2003)]. On the other hand, it is reported that Bcr-Abl is decomposed by treatment with an Hsp90 inhibitor since Bcr-Abl is a client protein for Hsp90 [Blood, 96, 2284-2291 (2000)]. It is also reported that geldanamycin or its derivative 17-allylamino-17-demethoxygeldanamycin (17-AAG) shows inhibitory activity against the growth of the Ba/F3 cell line, in which the mutant Bcr-Abl (point mutation of T315I or E255K) has been expressed to give an autonomously multiplying ability, by inducing decomposition of the mutant Bcr-Abl [Blood, 100, 3041-3044 (2002)]. Thus, the 17-AAG has been suggested to be effective in treating a patient of chronic myeloid leukemia resistant to imatinib.

In breast cancer, a cancer gene, her2/neu gene, is recognized to be amplified, and excess expression of a receptor- type tyrosine kinase Her2 has been introduced. Therefore, in the breast cancer in which Her2 has been expressed excessively, the growth of cells, the anchorage-independent growth, and the accentuation of tumorigenesis have occurred. High expression of Her2 is reported in 25-30% of the entire breast cancer, and the expression of Her2 is a factor of unfavorable prognosis [Science, 235, 177-182 (1987); Int. J. Cancer, 49, 650-655]. It is reported that a human antibody trastuzumab (Herceptin) which binds specifically the extra-cellular domain of such Her2 can be applied clinically to patients suffering from breast cancer expressing Her2 to exhibit a high anti-tumor effect. It is reported, however, that though a single medication is effective in only 30-40% of patients and the effectiveness is increased in combination with a chemotherapeutic agent, in either case, a continuous regimen generates resistant cancers [Journal of Clinical Oncology, 17, 2639-2648 (1999); New England Journal of Medicine, 344, 783-792 (2001)]. As the mechanism

of resistance to trastuzumab, accentuation of PI3 kinase, increase of the expression of insulin-like growth factor-1 (IGF-1), etc. are pointed out, though details are unknown. It is reported that trastuzumab has no biological effect (formation of Her2 dimer, cell internalization) for the cell line JIMT-1 established clinically from the trastuzumab resistant patients, but the 17-AAG has an effect equal to that for the trastuzumab sensitive strain SK-BR3 [Immunology Letters, 104, 146-155 (2006)].

As tyrosine kinase inhibitors for an epidermal growth factor (EGF) receptor, a low-molecular compound, gefitinib (Iressa) or erlotinib (Tarceva), has been applied clinically. It is reported that though these agents are effective to a patient who has an active-form mutation (deletion of exon 19, point mutaiton L858R, etc.) in the EGF receptor among non-small cell lung cancers, a continuous regimen brings about a secondary mutation (point mutation T790M, etc.) in the EGF receptor to generate resistance. The secondary T790Mpoint mutation was recognized in 7 out of 16 non-small cell lung cancer patients who were responsive to gefitinib or erlotinib but not responsive during a continuous regimen [New England Journal of Medicine, 352, 786-792 (2005); Clinical Cancer Research, 12, 6494-6501 (2007)]. On the other hand, it is reported that geldanamycin induces decomposition of the EGF receptor to inhibit the cell growth for the cell line NCI-H1975 derived from non-small lung cancer which expresses the T790M point mutant EGF receptor [Cancer Research, 65, 6401-6408 (2005)]. It is suggested that the Hsp90 inhibitor is effective for non-small cell lung cancer resistant to EGF receptor tyrosine kinase inhibitors.

Imatinib has been applied clinically as a therapeutic agent for gastrointestinal stromal tumor (GIST), since it inhibits not only Bcr-Abl but also KIT (c-kit gene product) and platelet-derived growth factor (PDGF) receptors. GIST is a carcinoma which is developed through mutation of the KIT or PDGF receptor and their constant activation. It is reported that imatinib is effective in approximately 80% of GIST patients prescribed and the patients who have a mutation (point mutation or deletion of V560D, etc.) at the exon 11 of KIT (the area of parietal cell membrane) show a higher response to it than the patients who have a mutation at the exon 9 [Journal of Clinical Oncology, 21, 4342-4349 (2003)]. In addition, a continuous regimen results in generating a secondary mutation which makes imatinib ineffective, causing a clinical problem of imatinib resistance. The mutation involved in the imatinib resistance is recognized with high frequency in the ATP-binding pocket of KIT (V654A, T670I point mutation, etc.) or kinase activating loop (point mutation at C809G, D816H, D820A/E/G, N822K/Y, Y823D, etc.) [Journal of Clinical Oncology, 24, 4764-4774 (2006)]. For the patients who express mutant KIT (V654A, T670I, etc.) which is not inhibited by imatinib, sunitinib (Sutent) which inhibits the PDGF receptor and KIT is effective and has been applied clinically. Sunitinib, however, is reported to be ineffective to the PDGF receptor mutant (D842V) involved in imatinib resistance [Clinical Cancer Research, 12, 2622-2627 (2003)] . On the other hand, 17-AAG inhibits KIT through proteolysis because KIT is a client protein. Therefore, 17-AAG is also reported to be effective to the cell lines GIST430 (KIT V654A point mutation) and GSIT48 (KIT V560E, D820D point mutation) expressing the imatinib- resistant variant KIT [Cancer Research, 66, 9153-9161 (2006)].

A proteasome inhibitor, bortezomib (Velcade), has been approved as an agent for treating multiple myeloma and applied clinically. Bortezomib is effective to approximately 35% of refractory/recurrent multiple myeloma, but ineffective to approximately 40%. Additionally, even in the effective cases the resistance develops after about one year, and thus, the bortezomib resistance is regarded as a clinical problem [New England Journal of Medicine, 348, 2609-2617 (2003)]. The phase I trial on 17-AAG was performed for refractory/recurrent multiple myeloma, and reported that 17-AAG was effective to some of the patients. In 9 out of the 22 patients, stable disease (SD, a stop of the progress) or more effect was observed [Blood, 106, Abstract 361 (2005)]. In addition, the phase I trial on an Hsp90 inhibitor IPI-504 was performed for refractory/recurrent multiple myeloma. The SD was observed in only 3 out of the 17 cases as anti-tumor effect [Blood, 108, Abstract 3579 (2006)]. In the clinical trial with the above-mentioned Hsp90 inhibitor alone, a causal relationship between the Hsp90 inhibitor and the bortezomib resistance is unknown because the medication history of the patients for bortezomib is not clear (since there were no intention to get the bortezomib-resistant patients together). The phase II trial on the combination of 17-AAG and bortezomib has been conducted for the patients of refractory/recurrent multiple myeloma in the U.S.A. The anti-tumor effect was observed as partial response (partial remission) or more in 8 out of 23 cases. In this trial, however, there is a possibility that the anti-tumor effect of bortezomib alone was observed since the trial was not performed collectively for the bortezomib-resistant patients; thus, the effect of 17-AAG could not be evaluated exactly [Blood, 108, Abstract 406 (2006)].

There are some papers reporting the molecular mechanism involved in resistance to the Hsp90 inhibitors. Human leukemia cell line in which the gene of Bcl-2 or Bcl-xL has been introduced to highly express the Bcl-2 or Bcl-xL having an inhibiting function for apoptosis, exhibits a resistance to the apoptosis induced by an Hsp90 inhibitor 17-AAG in comparison with the cell line into which the gene has not been introduced (Non-patent document 1). The human leukemia cell line in which the gene of the molecular chaperone Hsp70 has been introduced to highly express Hsp70, exhibits a resistance to the apoptosis induced by 17-AAG in comparison with the cell line into which the gene has not been introduced (Non-patent document 2). Human solid cancer cell line in which the gene of the molecular chaperone Hsp27 has been introduced to highly express Hsp27, resists suppression of the growth induced by 17-AAG in a colony forming assay, in comparison with the cell line into which the gene has not been introduced (Non-patent document 3). From these reports, it is suggested that increase of the expression of Hsp70, Hsp27, and Bcl-2 in the cancer cells leads

to the resistance to 17-AAG. On the other hand, it is also reported that the apoptosis-inducing effect of an Hsp90 antagonist PU24FCl, etc., for the small cell lung cancer cell line, does not depend on the expressed amount of Hsp70, and the apoptosis-inducing effect of the Hsp90 antagonist for the same cell line is not influenced by the expressed amount of Bcl-2 (Non-patent document 4). Therefore, it is not possible to conclude that increase of the expression of Hsp70 and Bcl-2 in cancer cells leads to the resistance of Hsp90 antagonists.

The Hsp90 family protein inhibitors and antitumor agents which comprises, as an active ingredient, a compound used in the present invention are known (Patent documents. 1, 2 and 3).

Patent document No. 1: WO2005/000778 pamphlet
Patent document No. 2: WO2005/063222 pamphlet
Patent document No. 3: WO2006/088193 pamphlet
Non patent document No. 1: Blood, 102, No. 1, 269-275, (2003)
Non patent document No. 2: Cancer Research, 65, No. 22, 10536-10544, (2005)
Non patent document No. 3: Cancer Research, 66, No. 22, 10967-10975, (2006)
Non patent document No. 4: Nature Chemical Biology, 3, 498-507, (2007)

Disclosure of the Invention

Problems to be Solved by the Invention

An object of the present invention is to provide a therapeutic agent for a cancer resistant to a protease inhibitor which comprises, as an active ingredient, an Hsp90 family protein inhibitor, and the like.

Means for Solving the Problems

The present invention relates to the following (1) to (56).

(1) A therapeutic agent for a cancer resistant to a protease inhibitor which comprises, as an active ingredient, a Heat shock protein 90 (Hsp90) family protein inhibitor.

(2) The therapeutic agent according to (1) wherein the Hsp90 family protein inhibitor is a benzoyl compound represented by formula (I) :

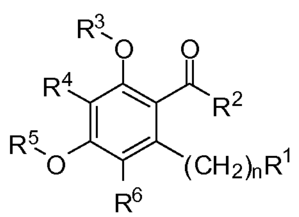

(I)

[wherein n represents an integer of 1 to 5;

$R^1$ represents substituted or unsubstituted lower alkyl, substituted or unsubstituted lower alkoxy, substituted or unsubstituted cycloalkyl, substituted or unsubstituted lower alkoxycarbonyl, substituted or unsubstituted heterocycle-alkyl, substituted or unsubstituted aryl, $CONR^7R^8$ (wherein $R^7$ and $R^8$, which may be the same or different, each represent a hydrogen atom, substituted or unsubstituted lower alkyl, substituted or unsubstituted cycloalkyl, substituted or unsubstituted lower alkanoyl, substituted or unsubstituted aryl, a substituted or unsubstituted heterocyclic group, substituted or unsubstituted aralkyl, substituted or unsubstituted heterocycle-alkyl, or substituted or unsubstituted aroyl, or $R^7$ and $R^8$ form a substituted or unsubstituted heterocyclic group together with the adjacent nitrogen atom thereto), or $NR^9R^{10}$ (wherein $R^9$ and $R^{10}$ have the same meanings as $R^7$ and $R^8$ defined above, respectively);

$R^2$ represents substituted or unsubstituted aryl, or a substituted or unsubstituted aromatic heterocyclic group;

$R^3$ and $R^5$, which may be the same or different, each represent a hydrogen atom, substituted or unsubstituted lower alkyl, substituted or unsubstituted lower alkenyl, substituted or unsubstituted lower alkanoyl, substituted or unsubstituted cycloalkyl, substituted or unsubstituted aralkyl, or substituted or unsubstituted aroyl;

$R^4$ represents a hydrogen atom, hydroxy or halogen; and

$R^6$ represents a hydrogen atom, halogen, cyano, nitro, substituted or unsubstituted lower alkyl, substituted or unsubstituted lower alkenyl, substituted or unsubstituted lower alkynyl, substituted or unsubstituted lower alkoxy,

substituted or unsubstituted cycloalkyl, amino, lower alkylamino, di-lower alkylamino, carboxy, substituted or unsubstituted lower alkoxycarbonyl, substituted or unsubstituted lower alkanoyl, substituted or unsubstituted aryloxy, substituted or unsubstituted aryl, a substituted or unsubstituted heterocyclic group, substituted or unsubstituted aralkyl, or substituted or unsubstituted heterocycle-alkyl] or a pharmaceutically acceptable salt thereof.

(3) The therapeutic agent according to the above (2) wherein $R^2$ is aryl substituted with 1 to 3 substituents or aryl.

(4) The therapeutic agent according to the above (2) wherein $R^2$ is phenyl substituted with 1 to 3 substituents or phenyl.

(5) The therapeutic agent according to the above (2) wherein $R^2$ is phenyl substituted with lower alkoxy and/or heterocycle-alkyloxy.

(6) The therapeutic agent according to the above (2) wherein $R^2$ is a substituted or unsubstituted aromatic heterocyclic group.

(7) The therapeutic agent according to any of the above (2) to (6) wherein $R^3$ and $R^5$, which may be the same or different, each are a hydrogen atom,substitutedor unsubstitutedlower alkyl,substituted or unsubstituted lower alkanoyl, substituted or unsubstituted aroyl, or substituted or unsubstituted lower alkenyl.

(8) The therapeutic agent according to any of the above (2) to (6) wherein $R^3$, $R^4$ and $R^5$ are hydrogen atoms.

(9) The therapeutic agent according to any of the above (2) to (8) wherein $R^1$ is $CONR^7R^8$ (wherein $R^7$ and $R^8$ have the same meanings as defined above, respectively).

(10) The therapeutic agent according to any of the above (2) to (8) wherein $R^1$ is $CONR^{7a}R^{8a}$ (wherein $R^{7a}$ and $R^{8a}$, which may be the same or different, each represent a hydrogen atom, substituted or unsubstituted lower alkyl, or substituted or unsubstituted heterocycle-alkyl).

(11) The therapeutic agent according to any of the above (2) to (8) wherein $R^1$ is $CONR^{7b}R^{8b}$ (wherein $R^{7b}$ and $R^{8b}$ form a substituted or unsubstituted heterocyclic group together with the adjacent nitrogen atom thereto).

(12) The therapeutic agent according to any of the above (2) to (8) wherein $R^1$ is $CONR^{7c}R^{8c}$ (wherein $R^{7c}$ and $R^{8c}$, which may be the same or different, each represent 2-hydroxyethyl or 2-methoxyethyl).

(13) The therapeutic agent according to any of the above (2) to (8) wherein $R^1$ is substituted or unsubstituted lower alkoxy.

(14) The therapeutic agent according to any of the above (2) to (13) wherein n is 1.

(15) The therapeutic agent according to any of the above (2) to (14) wherein $R^6$ is a hydrogen atom, lower alkyl, halogen or aryl.

(16) The therapeutic agent according to any of the above (2) to (14) wherein $R^6$ is lower alkyl.

(17) The therapeutic agent according to any of the above (2) to (14) wherein $R^6$ is ethyl.

(18) The therapeutic agent according to the above (1) wherein the Hsp90 family protein inhibitor is a benzoyl compound represented by formula (IA):

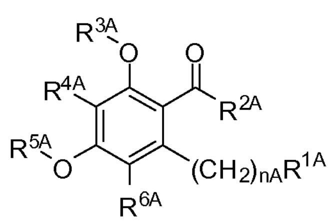

(IA)

[wherein nA represents an integer of 0 to 10;

$R^{1A}$ represents a hydrogen atom, hydroxy, cyano, carboxy, nitro, halogen, substituted or unsubstituted lower alkyl, substituted or unsubstituted lower alkenyl, substituted or unsubstituted lower alkynyl, substituted or unsubstituted lower alkoxy, substituted or unsubstituted cycloalkyl, substituted or unsubstituted lower alkoxycarbonyl, substituted or unsubstituted lower alkanoyloxy, substituted or unsubstituted heterocycle-alkyl, substituted or unsubstituted aryl, substituted or unsubstituted arylsulfonyl, a substituted or unsubstituted heterocyclic group, $CONR^7R^8$ (wherein $R^7$ and $R^8$ have the same meanings as defined above, respectively), or $NR^9R^{10}$ (wherein $R^9$ and $R^{10}$ have the same meanings as defined above, respectively);

$R^{2A}$ represents substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group;

$R^{3A}$ and $R^{5A}$, which may be the same or different, each represent a hydrogen atom, substituted or unsubstituted lower alkyl, substituted or unsubstituted lower alkenyl, substituted or unsubstituted lower alkanoyl, substituted or unsubstituted cycloalkyl, substituted or unsubstituted aralkyl or substituted or unsubstituted aroyl; and $R^{4A}$ and $R^{6A}$, which may be the same or different, each represent a hydrogen atom, hydroxy, halogen, cyano, nitro, substituted or unsubstituted lower alkyl, substituted or unsubstituted lower alkenyl, substituted or unsubstituted lower alkynyl,

substituted or unsubstituted lower alkoxy, substituted or unsubstituted cycloalkyl, amino, lower alkylamino, di-lower alkylamino, carboxy, substituted or unsubstituted lower alkoxycarbonyl, substituted or unsubstituted lower alkanoyl, substituted or unsubstituted aryloxy, substituted or unsubstituted aryl, a substituted or unsubstituted heterocyclic group, substituted or unsubstituted aralkyl, or substituted or unsubstituted heterocycle-alkyl] or a pharmaceutically acceptable salt thereof.

(19) The therapeutic agent according to the above (1) wherein the Hsp90 family protein inhibitor is a benzene derivative represented by formula (II):

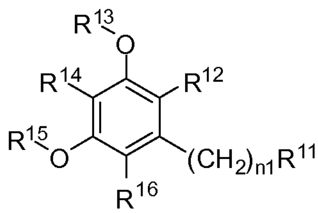

(II)

[wherein $n^1$ represents an integer of 0 to 10;

$R^{11}$ represents a hydrogen atom, hydroxy, cyano, carboxy, nitro, halogen, substituted or unsubstituted lower alkyl, substituted or unsubstituted lower alkenyl, substituted or unsubstituted lower alkynyl, substituted or unsubstituted cycloalkyl, substituted or unsubstituted lower alkoxycarbonyl, substituted or unsubstituted aroyl, substituted or unsubstituted lower alkanoyl, substituted or unsubstituted heterocycle-alkyl, substituted or unsubstituted aryl, substituted or unsubstituted aralkyl, substituted or unsubstituted arylsulfonyl, a substituted or unsubstituted heterocyclic group, $CONR^{17}R^{18}$ (wherein $R^{17}$ and $R^{18}$, which may be the same or different, each represent a hydrogen atom, substituted or unsubstituted lower alkyl, substituted or unsubstituted cycloalkyl, substituted or unsubstituted lower alkanoyl, substituted or unsubstituted aryl, a substituted or unsubstituted heterocyclic group, substituted or unsubstituted aralkyl, substituted or unsubstituted heterocycle-alkyl, or substituted or unsubstituted aroyl, or $R^{17}$ and $R^{18}$ form a substituted or unsubstituted heterocyclic group together with the adjacent nitrogen atom thereto), $NR^{19}R^{20}$ [wherein $R^{19}$ and $R^{20}$, which may be the same or different, each represent a hydrogen atom, substituted or unsubstituted lower alkylsulfonyl, substituted or unsubstituted lower alkyl, substituted or unsubstituted cycloalkyl, substituted or unsubstituted lower alkanoyl, substituted or unsubstituted aryl, a substituted or unsubstituted heterocyclic group, substituted or unsubstituted aralkyl, substituted or unsubstituted heterocycle-alkyl, substituted or unsubstituted aroyl, or $CONR^{21}R^{22}$ (wherein $R^{21}$ and $R^{22}$ have the same meanings as $R^{17}$ and $R^{18}$ defined above, respectively), or $R^{19}$ and $R^{20}$ form a substituted or unsubstituted heterocyclic group together with the adjacent nitrogen atom thereto], or $OR^{23}$ (wherein $R^{23}$ represents substituted or unsubstituted lower alkyl, substituted or unsubstituted lower alkenyl, substituted or unsubstituted lower alkanoyl, substituted or unsubstituted aryl, a substituted or unsubstituted heterocyclic group, substituted or unsubstituted aralkyl, or substituted or unsubstituted heterocycle-alkyl);

$R^{12}$ represents substituted or unsubstituted lower alkyl, substituted or unsubstituted lower alkenyl, substituted or unsubstituted lower alkynyl, substituted or unsubstituted aryl, or a substituted or unsubstituted heterocyclic group;

$R^{13}$ and $R^{15}$, which may be the same or different, each represent a hydrogen atom, substituted or unsubstituted lower alkyl, substituted or unsubstituted lower alkenyl, substituted or unsubstituted lower alkanoyl, substituted or unsubstituted cycloalkyl, substituted or unsubstituted lower alkylsulfonyl, substituted or unsubstituted arylsulfonyl, carbamoyl, sulfamoyl, substituted or unsubstituted lower alkylaminocarbonyl, substituted or unsubstituted di-lower alkylaminocarbonyl, substituted or unsubstituted lower alkoxycarbonyl, substituted or unsubstituted heterocycle-carbonyl, substituted or unsubstituted aralkyl, or substituted or unsubstituted aroyl; and

$R^{14}$ and $R^{16}$, which may be the same or different, each represent a hydrogen atom, hydroxy, halogen, cyano, nitro, substituted or unsubstituted lower alkyl, substituted or unsubstituted lower alkenyl, substituted or unsubstituted lower alkynyl, substituted or unsubstituted lower alkoxy, substituted or unsubstituted cycloalkyl, amino, lower alkylamino, di-lower alkylamino, carboxy, substituted or unsubstituted lower alkoxycarbonyl, substituted or unsubstituted aryloxy, substituted or unsubstituted aryl, a substituted or unsubstituted heterocyclic group, substituted or unsubstituted lower alkanoyl, substituted or unsubstituted aralkyl, or substituted or unsubstituted heterocycle-alkyl] or a pharmaceutically acceptable salt thereof.

(20) The therapeutic agent according to the above (19) wherein $R^{11}$ is a hydrogen atom, hydroxy, cyano, carboxy, nitro, halogen, substituted or unsubstituted lower alkyl, substituted or unsubstituted lower alkynyl, substituted or unsubstituted lower alkoxy, substituted or unsubstituted cycloalkyl, substituted or unsubstituted lower alkoxycarbonyl, substituted or unsubstituted lower alkanoyloxy, substituted or unsubstituted heterocycle-alkyl, substituted or unsubstituted aryl, substituted or unsubstituted arylsulfonyl, $CONR^{17}R^{18}$ (wherein, $R^{17}$ and $R^{18}$ have the same meanings as defined above, respectively), or $NR^{19}R^{20}$ (wherein, $R^{19}$ and $R^{20}$ have the same meanings as defined

above, respectively).

(21) The therapeutic agent according to the above (19) wherein $R^{11}$ is substituted or unsubstituted lower alkyl, substituted or unsubstituted lower alkynyl, substituted or unsubstituted lower alkoxy, substituted or unsubstituted cycloalkyl, substituted or unsubstituted lower alkoxycarbonyl, substituted or unsubstituted heterocycle-alkyl, substituted or unsubstituted aryl, $CONR^{17}R^{18}$ (wherein $R^{17}$ and $R^{18}$ have the same meanings as defined above, respectively), or $NR^{19}R^{20}$ (wherein $R^{19}$ and $R^{20}$ have the same meanings as defined above, respectively).

(22) The therapeutic agent according to any of the above (19) to (21) wherein $R^{12}$ is substituted or unsubstituted aryl, or a substituted or unsubstituted aromatic heterocyclic group.

(23) The therapeutic agent according to any of the above (19) to (21) wherein $R^{12}$ is substituted or unsubstituted aryl.

(24) The therapeutic agent according to any of the above (19) to (21) wherein $R^{12}$ is substituted or unsubstituted phenyl.

(25) The therapeutic agent according to any of the above (19) to (21) wherein $R^{12}$ is substituted or unsubstituted furyl.

(26) The therapeutic agent according to any of the above (19) to (25) wherein $R^{14}$ is a hydrogen atom, hydroxy or halogen.

(27) The therapeutic agent according to any of the above (19) to (26) wherein $R^{13}$ and $R^{15}$, which may be the same or different, each are a hydrogen atom, substituted or unsubstituted lower alkyl, substituted or unsubstituted lower alkenyl, substituted or unsubstituted lower alkanoyl, substituted or unsubstituted aroyl, substituted or unsubstituted lower alkylaminocarbonyl, substituted or unsubstituted di-lower alkylaminocarbonyl, substituted or unsubstituted lower alkoxycarbonyl, or substituted or unsubstituted heterocycle-carbonyl.

(28) The therapeutic agent according to any of the above (19) to (25) wherein $R^{13}$, $R^{14}$ and $R^{15}$ are hydrogen atoms.

(29) The therapeutic agent according to any of the above (1) to (28) wherein the cancer resistant to a protease inhibitor is cancer derived from hematopoietic tumor, breast cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, head and neck cancer, osteosarcoma, melanoma, or cancer derived from brain tumor resistant to a protease inhibitor.

(30) The therapeutic agent according to any of the above (1) to (28) wherein the cancer resistant to a protease inhibitor is leukemia, myeloma or lymphoma resistant to a protease inhibitor.

(31) The therapeutic agent according to any of the above (1) to (28) wherein the cancer resistant to a protease inhibitor is multiple myeloma resistant to a protease inhibitor.

(32) The therapeutic agent according to any of the above (1) to (28) wherein the cancer resistant to a protease inhibitor is a cancer in which expression of Hsp70, Hsp27 or Bcl-2 is increased.

(33) The therapeutic agent according to any of the above (1) to (32) wherein the protease inhibitor is an antibody having protease inhibiting activity.

(34) The therapeutic agent according to any of the above (1) to (32) wherein the protease inhibitor is a low-molecular compound having protease inhibiting activity.

(35) The therapeutic agent according to any of the above (1) to (34) wherein the protease inhibitor is a matrix metalloprotease inhibitor.

(36) The therapeutic agent according to any of the above (1) to (34) wherein the protease inhibitor is an inhibitor for urokinase plasminogen activator.

(37) The therapeutic agent according to any of the above (1) to (34) wherein the protease inhibitor is a cathepsin inhibitor.

(38) The therapeutic agent according to any of the above (1) to (34) wherein the protease inhibitor is a proteasome inhibitor.

(39) The therapeutic agent according to any of the above (1) to (34) wherein the protease inhibitor is an aminopeptidase inhibitor.

(40) The therapeutic agent according to the above (38) wherein the proteasome inhibitor is bortezomib.

(41) The therapeutic agent according to the above (38) wherein the proteasome inhibitor is MG-132.

(42) A method for treating a cancer resistant to a protease inhibitor, which comprises a step of adminstering an Hsp90 family protein inhibitor to a patient in need thereof.

(43) The method according to the above (42) wherein the Hsp90 family protein inhibitor is the benzoyl compound described in any of the above (2) to (17) or a pharmaceutically acceptable salt thereof.

(44) The method according to the above (42) wherein the Hsp90 family protein inhibitor is the benzoyl compound described in the above (18) or a pharmaceutically acceptable salt thereof.

(45) The method according to the above (42) wherein the Hsp90 family protein inhibitor is the benzene derivative described in any of the above (19) to (28) or a pharmaceutically acceptable salt thereof.

(46) The method according to any of the above (42) to (45) wherein the cancer resistant to a protease inhibitor is the cancer described in any of the above (29) to (32).

(47) The method according to any of the above (42) to (46) wherein the protease inhibitor is the protease inhibitor

described in any of the above (33) to (41).

(48) Use of an Hsp90 family protein inhibitor for the manufacture of a therapeutic agent for a cancer resistant to a protease inhibitor.

(49) The use according to the above (48) wherein the Hsp90 family protein inhibitor is the benzoyl compound described in any of the above (2) to (17) or a pharmaceutically acceptable salt thereof.

(50) The use according to the above (48) wherein the Hsp90 family protein inhibitor is the benzoyl compound described in the above (18) or a pharmaceutically acceptable salt thereof.

(51) The use according to the above (48) wherein the Hsp90 family protein inhibitor is the benzene derivative described in any of the above (19) to (28) or a pharmaceutically acceptable salt thereof.

(52) The use according to any of the above (48) to (51) wherein the cancer resistant to a protease inhibitor is the cancer described in any of the above (29) to (32).

(53) The use according to any of the above (48) to (52) wherein the protease inhibitor is the protease inhibitor described in any of the above (33) to (41).

(54) A therapeutic agent for a cancer in which expression of Hsp70, Hsp27 or Bcl-2 is increased, which comprises, as an active ingredient, the benzoyl compound or the benzene derivative described in any of the above (2) to (28) or a pharmaceutically acceptable salt thereof as the Hsp90 family protein inhibitor.

(55) A method for treating a cancer in which expression of Hsp70, Hsp27 or Bcl-2 is increased, which comprises, a step of administering the Hsp90 family protein inhibitor comprising, as an active ingredient, the benzoyl compound or the benzene derivative described in any of the above (2) to (28) or a pharmaceutically acceptable salt thereof to a patient in need thereof.

(56) Use of an Hsp90 family protein inhibitor which comprises, as an active ingredient, the benzoyl compound or the benzene derivative described in any of the above (2) to (28) or a pharmaceutically acceptable salt thereof for the manufacture of a therapeutic agent for a cancer in which expression of Hsp70, Hsp27 or Bcl-2 is increased.

## Effect of the Invention

The present invention provides a therapeutic agent for a cancer resistant to a protease inhibitor which comprises, as an active ingredient, an Hsp90 family protein inhibitor, and the like.

## Brief Description of the Drawings

Fig. 1 shows the growth inhibitory effect of MG-132 to KMS-11 and KMS-11/Bor. The ordinate indicates % control of living cell. The abscissa indicates compound concentration. White circles denote growth inhibitory effect on KMS-11 cells; and black circles denote growth inhibitory effect on KMS-11/Bor.

Fig. 2 shows the growth inhibitory effect of bortezomib to KMS-11 and KMS-11/Bor. The ordinate indicates % control of living cell. The abscissa indicates compound concentration. White circles denote the growth inhibitory effect on KMS-11 cells; and black circles denote growth inhibitory effect on KMS-11/Bor.

Fig. 3 shows growth inhibitory effect of 17-AAG to KMS-11 and KMS-11/Bor. The ordinate indicates % control of living cell. The abscissa indicates compound concentration. White circles denote the growth inhibitory effect on KMS-11 cells; and black circles denote growth inhibitory effect on KMS-11/Bor.

Fig. 4 shows growth inhibitory effect of Compound 22 to KMS-11 and KMS-11/Bor. The ordinate indicates % control of living cell. The abscissa indicates compound concentration. White circles denote the growth inhibitory effect on KMS-11 cells; and black circles denote growth inhibitory effect on KMS-11/Bor.

Fig. 5 shows growth inhibitory effect of MG-132 to OPM-2 and OPM-2/Bor. The ordinate indicates % control of living cell. The abscissa indicates compound concentration. White circles denote growth inhibitory effect of each compound to OPM-2 cells and black circles denote inhibitory effect on OPM-2/Bor.

Fig. 6 shows growth inhibitory effect of bortezomib to OPM-2 and OPM-2/Bor. The ordinate indicates % control of living cell. The abscissa indicates compound concentration. White circles denote growth inhibitory effect of each compounds to OPM-2 cells; and black circles denote inhibitory effect on OPM-2/Bor.

Fig. 7 shows growth inhibitory effect of 17-AAG to OPM-2 and OPM-2/Bor. The ordinate indicates % control of living cell. The abscissa indicates compound concentration. White circles denote growth inhibitory effect of each compounds to OPM-2 cells; and black circles denote inhibitory effect on OPM-2/Bor.

Fig. 8 shows growth inhibitory effect of Compound 22 to OPM-2 and OPM-2/Bor. The ordinate indicates % control of living cell. The abscissa indicates compound concentration. White circles denote growth inhibitory effect of each compounds to OPM-2 cells; and black circles denote inhibitory effect on OPM-2/Bor.

Fig. 9 shows antitumor effect of Compound 22 and bortezomib to OPM-2/Bor cell transplant mouse model. The

ordinate indicates the ratio of change in tumor volume (V/V0) based on the tumor volume at day 0 (V0), and the abscissa indicates days. Black circles denotes growth inhibitory effect when test compound is not administered; black triangles denote growth inhibitory effect of 1 mg/kg administration of bortezomib; white triangles denote growth inhibitory effect of 0.5 mg/kg administration of bortezomib; black diamonds denote growth inhibitory effect of 100 mg/kg administration of Compound 22; white diamond denote growth inhibitory effect of 50 mg/kg administration of Compound 22.

Fig. 10 shows expression level of Hsp70, Hsp27, Bcl-2 or β-Actin on OPM-2 or OPM-2/Bor.

## Best Modes for Carrying Out the Invention

Hereinafter, the compounds represented by formulae (I), (IA) and (II) are referred to as Compounds (I), (IA), (II) and the same applies to compounds of other formula numbers.

In the definitions of the groups in formula (I), (IA) and (II) :

Examples of the lower alkyl and the lower alkyl moieties of the lower alkoxy, lower alkoxycarbonyl, lower alkylamino, di-lower alkylamino, lower alkylaminocarbonyl, di-lower alkylaminocarbonyl and lower alkylsulfonyl include straight-chain or branched alkyl having 1 to 8 carbon atoms, such as methyl, ethyl, propyl, isopropyl, butyl, sec-butyl, tert-butyl, pentyl, isopentyl, neopentyl, hexyl, heptyl, and octyl. The two lower alkyl moieties of the di-lower alkylamino and di-lower alkylaminocarbonyl may be the same or different.

Examples of the lower alkenyl include straight-chain or branched alkenyl having 2 to 8 carbon atoms, such as vinyl, allyl, 1-propenyl, methacryl, crotyl, 1-butenyl, 3-butenyl, 2-pentenyl, 4-pentenyl, 2-hexenyl, 5-hexenyl, 2-heptenyl and 2-octenyl.

Examples of the lower alkynyl include straight-chain or branched alkynyl having 2 to 8 carbon atoms, such as ethynyl, propynyl, butynyl, pentynyl, hexynyl, heptynyl and octynyl.

Examples of the lower alkanoyl and the lower alkanoyl moiety of the lower alkanoyloxy include straight-chain or branched alkanoyl having 1 to 7 carbon atoms, such as formyl, acetyl, propionyl, butyryl, isobutyryl, valeryl, isovaleryl, pivaloyl, hexanoyl and heptanoyl.

Examples of the cycloalkyl include cycloalkyl having 3 to 8 carbon atoms, such as cyclopropyl, cyclobutyl, cyclopentyl, cyclohexyl, cycloheptyl and cyclooctyl.

Examples of the aryl and the aryl moieties of the arylsulfonyl, aryloxy and aroyl include monocyclic, bicyclic or tricyclic aryl having 6 to 14 carbon atoms, such as phenyl, indenyl, naphthyl and anthryl.

Examples of the aralkyl include aralkyl having 7 to 15 carbon atoms, such as benzyl, phenethyl, benzhydryl and naphthylmethyl.

Examples of the aromatic heterocyclic group include 5- or 6-membered monocyclic aromatic heterocyclic groups containing at least one atom selected from a nitrogen atom, an oxygen atom and a sulfur atom, and bicyclic or tricyclic condensed aromatic heterocyclic groups containing at least one atom selected from a nitrogen atom, an oxygen atom and a sulfur atom in which 3- to 8-membered rings are condensed, such as pyridyl, pyrazinyl, pyrimidinyl, pyridazinyl, quinolinyl, isoquinolinyl, phthalazinyl, quinazolinyl, quinoxalinyl, naphthyridinyl, cinnolinyl, pyrrolyl, pyrazolyl, imidazolyl, triazolyl, tetrazolyl, thienyl, furyl, thiazolyl, oxazolyl, indolyl, indazolyl, benzimidazolyl, benzotriazolyl, benzothiazolyl, benzoxazolyl, purinyl and benzodioxolanyl.

Examples of the heterocyclic group and the heterocyclic moieties of the heterocycle-alkyl, heterocycle-alkyloxy and heterocycle-carbonyl include groups described in the above definition of the aromatic heterocyclic group and also aliphatic heterocyclic groups. Examples of the aliphatic heterocyclic group include 5- or 6-membered monocyclic aliphatic heterocyclic groups containing at least one atom selected from a nitrogen atom, an oxygen atom and a sulfur atom, and bicyclic or tricyclic condensed aliphatic heterocyclic groups containing at least one atom selected from a nitrogen atom, an oxygen atom and a sulfur atom in which 3- to 8-membered rings are condensed, such as pyrrolidinyl, piperidino, piperazinyl, piperazinyl, morpholino, morpholinyl, thiomorpholino, thiomorpholinyl, homopiperidino, homopiperazinyl, homopiperazinyl, tetrahydropyridinyl, tetrahydroquinolinyl, tetrahydroisoquinolinyl, tetrahydrofuranyl, tetrahydropyranyl, dihydrobenzofuranyl, oxopiperazinyl, 2-oxopyrrolidinyl, oxolanyl, dioxolanyl.

Examples of the heterocyclic group formed together with the adjacent nitrogen atom include 5- or 6-membered monocyclic heterocyclic groups containing at least one nitrogen atom (the monocyclic heterocyclic groups may also contain another nitrogen atom, an oxygen atom or a sulfur atom), and bicyclic or tricyclic condensed heterocyclic groups containing at least one nitrogen atom in which 3- to 8-membered rings are condensed (the condensed heterocyclic groups may also contain another nitrogen atom, an oxygen atom or a sulfur atom), such as pyrrolidinyl, piperidino, piperazinyl, morpholino, thiomorpholino, homopiperidino, homopiperazinyl, tetrahydropyridinyl, tetrahydroquinolinyl, tetrahydroisoquinolinyl, oxopiperazinyl and 2-oxopyrrolidinyl.

The alkylene moieties of the heterocycle-alkyl and heterocycle-alkyloxy have the same meanings as a group

produced by removing one hydrogen atom from the lower alkyl defined above.

The halogen means each atoms of fluorine, chlorine, bromine and iodine.

Examples of the substituents (A) in the substituted lower alkyl, substituted lower alkoxy, substituted lower alkoxycarbonyl, substituted lower alkylaminocarbonyl, lower alkyl (substituted lower alkyl)aminocarbonyl, di-(substituted lower alkyl)aminocarbonyl, substituted lower alkylsulfonyl, substituted lower alkenyl and substituted lower alkynyl, include 1 to 3 substituents which may be the same or different, such as hydroxy, oxo, cyano, nitro, carboxy, amino, halogen, substituted or unsubstituted lower alkoxy, cycloalkyl, lower alkanoyl, lower alkoxycarbonyl, lower alkylamino, di-lower alkylamino, and hydroxy-lower alkylaminocarbonyl. The position(s) to be substituted with the substituent (s) is/are not particularly limited. Here, the halogen, lower alkoxy, cycloalkyl, lower alkanoyl, lower alkoxycarbonyl, lower alkylamino and di-lower alkylamino have the same meanings as defined above, respectively. The alkylene moiety of the hydroxy-lower alkylaminocarbonyl has the same meaning as a group produced by removing one hydrogen atom from the lower alkyl defined above. Examples of the substituents in the substituted lower alkoxy include 1 to 3 substituents which may be the same or different, such as hydroxy and halogen, and the halogen has the same meaning as defined above. The two lower alkyl moieties of the di-lower alkylamino may be the same or different.

Examples of the substituents (B) in the substituted lower alkanoyl, substituted lower alkanoyloxy, substituted cycloalkyl, substituted aryl, substituted arylsulfonyl, substituted aryloxy, substituted aralkyl, substituted aroyl, substituted phenyl, a substituted heterocyclic group, substituted heterocycle-alkyl, substituted furyl, substituted heterocycle-carbonyl, substituted aromatic heterocyclic group and substituted heterocyclic group formed together with the adjacent nitrogen atom include 1 to 3 substituents which may be the same or different, such as hydroxy, halogen, nitro, cyano, amino, carboxy, carbamoyl, substituted or unsubstituted lower alkyl, substituted or unsubstituted lower alkenyl, lower alkylaminocarbonyl, di-lower alkylaminocarbonyl, substituted or unsubstituted lower alkoxy, aralkyloxy, lower alkylsulfonyl, lower alkylsulfanyl, lower alkylthio, aryloxy, cycloalkyl, lower alkoxycarbonyl, lower alkylamino, di-lower alkylamino, lower alkanoyl , heterocyclic group, substituted or unsubstituted aryl, substituted or unsubstituted heterocycle-alkyloxy, substituted or unsubstituted heterocycle-carbonylalkyloxy. The position(s) to be substituted with substituent(s) is/are not particularly limited. Here, the halogen, lower alkyl, lower alkenyl, lower alkylaminocarbonyl, di-lower alkylaminocarbonyl, lower alkoxy, aryloxy, cycloalkyl, lower alkoxycarbonyl, lower alkylamino, di-lower alkylamino, lower alkanoyl , heterocyclic group and aryl have the same meanings as defined above, respectively; the lower alkyl moieties of the lower alkylsulfonyl, lower alkylsulfanyl and lower alkylthio have the same meaning as the lower alkyl defined above; the aralkyl moiety of the aralkyloxy has the same meaning as the aralkyl defined above; and the heterocyclic moiety and the alkylene moiety of the heterocycle-alkyloxy and heterocycle-carbonylalkyloxy have the same meanings as the heterocyclic group defined above, and the group produced by removing a hydrogen atom from the lower alkyl defined above. Examples of the substituents in the substituted lower alkyl, substituted lower alkenyl , substituted lower alkoxy and substituted aryl include 1 to 3 substituents which may be the same or different, such as hydroxy, carboxy, lower alkanoyl, halogen, lower alkoxy, cyano, lower alkylamino, di-lower alkylamino (the two lower alkyl moieties of the di-lower alkylamino may be the same or different) and the halogen, lower alkanoyl, lower alkoxy, lower alkylamino and di-lower alkylamino have the same meanings as defined above, respectively. Examples of the substituents in the substituted heterocycle-alkyloxy and substituted heterocycle-carbonylalkyloxy include 1 to 3 substituents which may be the same or different, such as hydroxy, halogen, lower alkyl, lower alkoxy, and heterocyclic group. Herein, the halogen, lower alkyl, lower alkoxy and heterocyclic group have the same meanings as defined above, respectively.

The pharmaceutically acceptable salts of Compounds (I), (IA) and (II) include pharmaceutically acceptable acid addition salts, metal salts, ammonium salts, organic amine addition salts and amino acid addition salts.

Examples of the pharmaceutically acceptable acid addition salts of Compounds (I), (IA) and (II) include inorganic acid addition salts such as hydrochloride, sulfate, nitrate and phosphate, and organic acid addition salts such as acetate, maleate, fumarate and citrate. Examples of the pharmaceutically acceptable metal salts include alkali metal salts such as sodium salt and potassium salt, alkaline earth metal salts such as magnesium salt and calcium salt, aluminum salt, and zinc salt. Examples of the pharmaceutically acceptable ammonium salts include salts of ammonium and tetramethylammonium. Examples of the pharmaceutically acceptable organic amine addition salts include an addition salt of morpholine or piperidine. Examples of the pharmaceutically acceptable amino acid addition salts include an addition salt of glycine, phenylalanine, lysine, aspartic acid or glutamic acid.

To obtain salt of Compounds (I), (IA) and (II), Compounds (I), (IA) and (II) can be purified as they are when they are obtained in a form of salt. When they are obtained in a free form, Compounds (I), (IA) and (II) can be dissolved or suspended in an appropriate solvent and acid or base and the like are added thereto to form salt.

For some of Compounds (I), (IA) and (II), there may exist stereoisomers such as positional isomers, geometrical isomers and optical isomers, and all possible isomers including these and mixtures thereof in any ratio can be used for the therapeutic agent of the present invention.

Further, Compounds (I), (IA), (II) and pharmaceutically acceptable salts thereof may exist in the form of adducts with water or various solvents, and these adducts can also be used as the therapeutic agent of the present invention.

The term "inhibition of Hsp90 family protein" refers to inhibition of the binding of Hsp90 family protein to a

protein to which Hsp90 family protein binds (Hsp90 client protein).

Examples of Hsp90 family proteins include Hsp90α protein, Hsp90β protein, grp94 and Hsp75/TRAP1.

The proteins to which Hsp90 family proteins bind include any proteins to which Hsp90 family proteins bind, for example, EGF receptor, Erb-B2, Bcr-Abl, src, raf-1, AKT, Flt-3, PLK, Wee1, FAK, cMET, hTERT, HIF1-α, mutant p53, estrogen receptors and androgen receptors [Expert Opinion on Biological Therapy, 2, 3-24, (2002)].

Examples of Hsp90 familyprotein inhibitor include, for example, Radicicol, Geldanamycin, 17-AAG, Herbimycin A, Novobiocin, Compounds (I), (IA), (II) and the like.

Hsp70 family proteins are the general term for Hsp having the molecular weight of 70 kDa or so, and examples thereof include Hsp70, Hsc70, Bip/Grp78, mtHsp70 and the like.

Hsp27 family proteins are the general term for the Hsp having the molecular weight 27 kDa or so, and Hsp27 protein is one of low molecular Hsp's.

Bcl-2 family proteins are a class of proteins which play an important role in controlling apoptosis.

In a number of therapeutic agents for cancers, it is known that cancer cells acquire resistance to said agents during long term administration. Sometimes observed is a phenomenon that the effect recognized at the early stage of therapy decreases during continuous medication or no effect is observed in a case of recurrence. Therefore, therapeutic agents effective in treating the cancers resistant to anti-tumor agents, including, for example, agents effective to multiple myeloma resistant to protease inhibitors, are needed.

Proteases (proteolytic enzymes) are enzymes which hydrolyze the peptide linkage of proteins or peptides, and are known to have a variety of physiological activities.

Protease inhibitors include, for example, matrix metalloprotease inhibitor, urokinase plasminogen activator inhibitor, cathepsin inhibitor, proteasome inhibitor, aminopeptidase inhibitor, and the like.

Examples of the matrix metalloprotease inhibitor include, for example, Batimastat (BB-94), Ilomastat (GM-6001), Marimastat (BB-2516), Tanomastat (BAY-12-9566), Prinomastat (AG-3340), Metastat (COL-3), Neovastat (AE-941), BMS-275291, MMI-270B (CGS-27023A), Trocade (Ro-32-3555), MMI-166 and the like [Nature Reviews Cancer, 6, 227, (2006)].

Examples of the urokinase plasminogen activator inhibitor include, for example, WX-771 (WO2007/025718), WX-671 [Expert Opinion on Biological Therapy, 6, 257, (2006)], WX-UK-1 [Neoplasma, 52, 185, (2005)], B-428 [Neoplasma, 52, 185, (2005)] and the like.

Examples of the cathepsin inhibitor include, for example, AFG-495 (AACR Annual Meeting, abstract 2910, 2005), E-64, CA030, CA074, NS-134, CLIK148 [literature of E-64, CA030, CA074, NS-134 and CLIK148: Biochemical Journal, 381, 511, (2004)] and the like.

Examples of the proteasome inhibitor [European Journal of Cancer, 42, 1623, (2006)] include, for example, bortezomib, MG-132, carfilzomib (PR-171, US2005/0245435), NPI-0052 [British Journal of Cancer, 95, 961, (2006)], SC-68896 (WO2007/017284), tyropeptin A, TP-110, TP-104 (WO2005/105826), belactosin derivative [Biochemical Pharmacology, 67, 227, (2004)] and the like.

Examples of the aminopeptidase inhibitor include, for example, ubenimex (Bestatin), TNP-470 [Angiogenesis, 7, 91, (2004)], PPI-2458 [Clinical Cancer Research, 12, 2583, (2006)], CHR-2797 [Proceedings of the American Society for Clinical Oncology, 25, abstract 3053, (2006)] and the like.

Compounds (I) and (IA) or a pharmaceutically acceptable salt thereof used in the present invention can be synthesized based on the method described in, for example, WO2005/000778.

Compound (II) or a pharmaceutically acceptable salt thereof used in the present invention can be synthesized based on the method described in, for example, WO2005/063222.

Specific examples of the compounds used in the present invention will be described in the following Tables 1 and 2, however the present invention is not limited to them. In the following tables, Ph represents phenyl.

Compounds 1 to 22 described in Table 1 can be synthesized by a method described in WO2005/000778. Compounds 23 to 37 described in Table 2 can be synthesized by a method described in WO2005/063222.

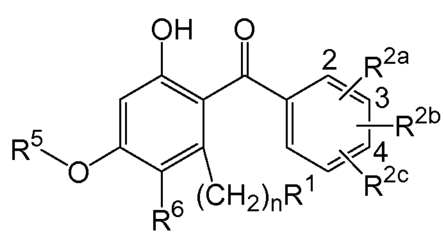

(I-i)

Table 1-1

| Compound | $R^1$ | n | $R^{2a}$ | $R^{2b}$ | $R^{2c}$ | $R^5$ | $R^6$ |
|---|---|---|---|---|---|---|---|
| 1 | $OCH_3$ | 2 | H | H | H | H | H |
| 2 | $OCH_3$ | 2 | H | H | H | H | Br |
| 3 | $OCH_3$ | 2 | H | H | H | H | $COCH_3$ |
| 4 | $CO_2CH_3$ | 1 | 3-$OCH_3$ | H | H | H | $CH_2CH_3$ |
| 5 | $OCH_3$ | 2 | 4-$OCH_3$ | H | H | H | $CH_2CH_3$ |
| 6 | $OCH_3$ | 2 | 4-$NO_2$ | H | H | H | $CH_2CH_3$ |
| 7 | $OCH_2CH_2OCH_3$ | 2 | 4-$OCH_3$ | H | H | H | $CH_2CH_3$ |
| 8 | $CON(CH_3)CH_2CH_2OH$ | 1 | 4-$OCH_3$ | H | H | H | $CH_2CH_3$ |
| 9 | 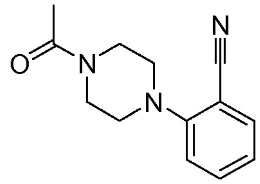 | 1 | 4-$OCH_3$ | H | H | H | $CH_2CH_3$ |
| 10 | $CO_2CH_3$ | 1 | 4-$OCH_3$ | H | H | $CH_2CH=CH_2$ | H |
| 11 | 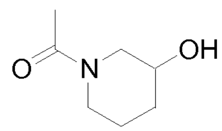 | 1 | 4-$OCH_3$ | H | H | H | $CH_2CH_3$ |
| 12 | 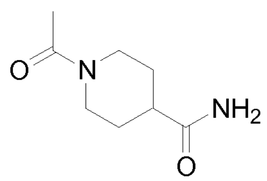 | 1 | 4-$OCH_3$ | H | H | H | $CH_2CH_3$ |

Table 1-2

| Compound | $R^1$ | n | $R^{2a}$ | $R^{2b}$ | $R^{2c}$ | $R^5$ | $R^6$ |
|---|---|---|---|---|---|---|---|
| 13 | 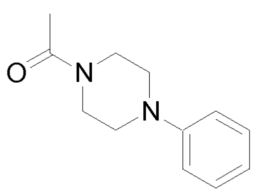 | 1 | 4-$OCH_3$ | H | H | H | $CH_2CH_3$ |
| 14 | 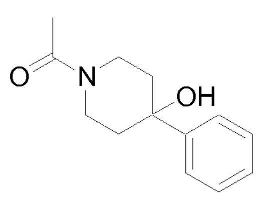 | 1 | 4-$OCH_3$ | H | H | H | $CH_2CH_3$ |
| 15 | $OCH_2CH(OH)CH_2OH$ | 2 | 2-F | 4-$OCH_3$ | H | H | $CH_2CH_3$ |

(continued)

| Compound | $R^1$ | n | $R^{2a}$ | $R^{2b}$ | $R^{2c}$ | $R^5$ | $R^6$ |
|---|---|---|---|---|---|---|---|
| 16 | 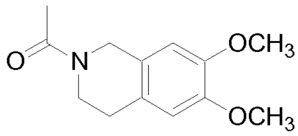 | 1 | 4-$OCH_3$ | H | H | H | $CH_2CH_3$ |
| 17 | $OCH_2CH(OH)CH_2OH$ | 2 | 3 - 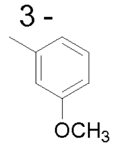 | 4-$OCH_3$ | H | H | $CH_2CH_3$ |
| 18 | $CON(CH_2CH_2OH)_2$ | 1 | 4-$OCHF_2$ | H | H | H | $CH_2CH_3$ |
| 19 | $CON(CH_2CH_2OH)CH_2CH_2OCH_3$ | 1 | 4-$SCH_3$ | H | H | H | $CH_2CH_3$ |
| 20 | $CON(CH_2CH_2OH)_2$ | 1 | 4-$SO_2CH_3$ | H | H | H | $CH_2CH_3$ |
| 21 | $CON(CH_2CH_2OH)CH_2CH_2OCH_3$ | 1 | 3- 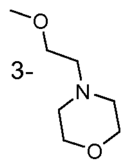 | 4-$OCH_3$ | H | H | $CH_2CH_3$ |
| 22 | $CON(CH_2CH_2OCH_3)_2$ | 1 | 3-$OCH_3$ | 4- 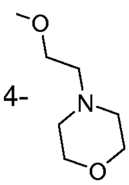 | H | H | $CH_2CH_3$ |

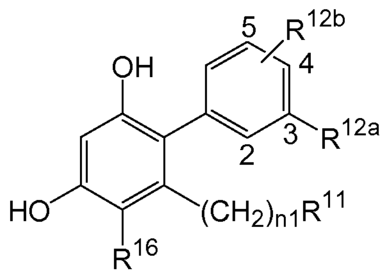

(II-i)

Table 2

| compound | $R^{11}$ | n1 | $R^{12a}$ | $R^{12b}$ | $R^{16}$ |
|---|---|---|---|---|---|
| 23 | $CO_2CH_3$ | 1 | H | H | H |
| 24 | $CO_2CH_3$ | 1 | $CH{=}CHCOCH_3$ | H | H |
| 25 | $CO_2CH_3$ | 1 | $(CH_2)\ _2COCH_3$ | H | H |
| 26 | $CO_2CH_3$ | 1 | $COCH_3$ | H | H |
| 27 | $CONH(CH_2)_2N(CH_3)_2$ | 1 | H | H | Br |

(continued)

| compound | $R^{11}$ | n1 | $R^{12a}$ | $R^{12b}$ | $R^{16}$ |
|---|---|---|---|---|---|
| 28 | 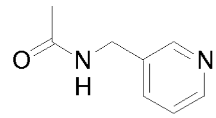 | 1 | H | H | Br |
| 29 | 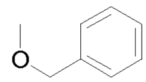 | 1 | H | H | Br |
| 30 | $CONH_2$ | 0 | H | H | Br |
| 31 | $CH=CHCO_2CH_3$ | 0 | H | H | Br |
| 32 | OH | 0 | H | H | Br |
| 33 | $CO_2CH_3$ | 1 | $CH_2CH_2CO_2H$ | H | Br |
| 34 | $CO_2CH_3$ | 1 | H | H | $CH_2Ph$ |
| 35 | $CO_2CH_3$ | 1 | H | 4-OPh | H |
| 36 | $OCH_2CONHCH_2CH_2OH$ | 3 | H | H | $CH_2CH_3$ |
| 37 | $OCH_2CH(OH)CH_2OH$ | 2 | 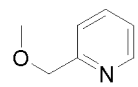 | H | $CH_2CH_3$ |

The therapeutic agents of the present invention can be used in the treatment of a cancer resistant to a protease inhibitor. For example, they can be used in treatment of cancer derived from hematopoietic tumor (for example, leukemia such as acute myeloid leukemia, chronic myeloid leukemia, acute lymphocytic leukemia, or chronic lymphocytic leukemia; myeloma such as multiple myeloma; or lymphoma), breast cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, head and neck cancer, osteosarcoma, melanoma, or cancer derived from brain tumor resistant to a protease inhibitor. In particular, it is preferable to use them in treatment of leukemia, myeloma, or lymphoma resistant to a protease inhibitor.

On the other hand, the therapeutic agents of the present invention can be used in treatment of a cancer in which the expression of Hsp70, Hsp27 or Bcl-2 has been increased, and preferably they can be used in treatment of a cancer which has resistance to a protease inhibitor and in which the expression of Hsp70, Hsp27 or Bcl-2 has been increased. More preferably, they can be used in treatment of multiple myeloma, or the like, in which the expression of Hsp70, Hsp27 or Bcl-2 has been increased.

In the present invention, the pharmaceutical compositions which are effective to a cancer resistant to a protease inhibitor can be evaluated using a cultured cell line. In addition, the pharmaceutical compositions which are effective in treating a cancer in which the expression of Hsp70, Hsp27 or Bcl-2 has been increased can be evaluated using a cultured cell line.

The cultured cell line used in the present invention includes, for example, KMS-11 cells and OPM-2 cells which have allowed to acquire resistance to protease inhibitors. The KMS-11 cells and OPM-2 cells, which are human cells derived from patients of multiple myeloma, are available from public cell banks such as German Collection of Microorganisms and Cell Cultures, various research institutes, or are avilable commercially. The cell lines KMS-11/Bor and OPM-2/Bor which have acquired resistance to protease inhibitors were provided by adding a protease inhibitor bortezomib to a culture broth of the KMS-11 cells and OPM-2 cells, followed by incubating continuously. The KMS-11/Bor cells and OPM-2/Bor cells which have acquired resistance to protease inhibitors may become a model of cancer resistant to protease inhibitors, and more preferably a model of multiple myeloma resistant to protease inhibitors.

The effect of the pharmaceutical composition of the present invention may be examined by measuring an *in vitro* cell growth inhibiting activity or measuring an *in vivo* anti-tumor activity using an animal model. As for a method for measuring the *in vitro* cell growth inhibiting activity, an examination with a cancer cell line resistant to anti-tumor agents, for example, cancer cells resistant to protease inhibitors, is included. In an examination for measuring an *in vivo* anti-tumor activity using an animal model, an immunodeficient mouse such as nude mouse in which a cancer cell line resistant to anti-tumor agents, e.g., cancer cells resistant to protease inhibitors, has been implanted, may be employed.

In the present invention, a pharmaceutical composition for the Hsp90 family protein inhibitors useful in treatment of cancers resistant to protease inhibitors can be screened and selected by evaluating the *in vitro* cell growth inhibiting

activity of the Hsp90 family protein inhibitor with a culture cell line derived from cancer tissues resistant to protease inhibitors, or alternatively by evaluating the administration of the Hsp90 family protein inhibitor using an animal model.

In the present invention, a pharmaceutical composition of the Hsp90 family protein inhibitors useful in treatment of cancers in which the expression of Hsp70, Hsp27 or Bcl-2 has increased, can be screened and selected by evaluating the *in vitro* cell growth inhibiting activity of the Hsp90 family protein inhibitor with a culture cell line derived from cancer tissues in which the expression of Hsp70, Hsp27 orBcl-2 has increased, or alternatively by evaluating the administration of the Hsp90 family protein inhibitor using an animal model.

The Compounds (I), (IA), (II) or pharmaceutically acceptable salts thereof *per se* may be administered alone, but usually it is desirable to provide them in various forms of pharmaceutical preparations.

The therapeutic agents of the present invention comprise, as an active ingredient, the Compounds (I), (IA), (II) or pharmaceutically acceptable salts thereof alone, or optionally in combination with any other therapeutically active ingredients. Those pharmaceutical preparations may be prepared according to any method well-known in the art of formulations by blending an active ingredient together with one or more of pharmaceutically acceptable carriers.

The route of administration is desirably chosen so as to be the most effective in therapeutic treatment, and oral administration or parenteral administration such as intravenous administration is included.

These formulations may be prepared in a conventional way by using, in addition to the active ingredient, pharmaceutically acceptable diluents, excipients, disintegrators, lubricants, binders, surfactants, water, physiological saline, vegetable oil, solubilizing agents, isotonizing agents, preservatives, anti-oxidants, or the like.

In preparing tablets, for example, excipients such as lactose, disintegrators such as starch, lubricants such as magnesium stearate, binders such as hydroxypropylcellulose, surfactants such as fatty acid esters, or plasticizers such as glycerin, may be used in a conventional way.

In preparing injections, water, physiological saline, vegetable oil, solvents, solubilizing agents, isotonizing agents, preservatives, anti-oxidants, or the like may be used in a conventional way.

The Compounds (I), (IA), and (II) or pharmaceutically acceptable salts thereof, when used in the above-mentioned object, mayusuallybe administered orally or parenterally such as injections, and the effective dose and frequency of administration depend on the form of administration, the age, body weight, and condition of the patient; and usually they may be administered at a daily dose of 0.01-20 mg/kg.

Subsequently, the effect of the therapeutic agents of the present invention will be explained specifically by the following Test Examples. In the following Test Examples, bortezomib and MG-132 were used as protease inhibitor, and 17-AAG and Compound 22 as Hsp90 inhibitor as a test compound. The Hsp90 inhibitory activity of Compound 22 is described in W0 2005/000778.

<u>Test Example 1: Preparation of KMS-11 cells and OPM-2 cells which have acquired bortezomib resistance</u>

KMS-11 cells were suspended in an RPMI1640 medium containing 10% fetal calf serum (FCS) at a concentration of $5 \times 10^4$ cells/mL, and incubated at 37˚C in a 5% carbon dioxide incubator. Then, bortezomib was added thereto to adjust the final concentration of 5 nmol/L, and the incubation was continued. After confirmation of the survival and satisfactory growth of the cells, the cells were suspended at a concentration of $5 \times 10^4$ cells/mL, then bortezomib was added at a concentration of 10 nmol/L, and the incubation was continued. Then, after confirmation of the survival and satisfactory growth of the cells, the concentration of bortezomib was increased step by step, and the culture and subculture were repeated to create the bortezomib-resistant KMS-11 cells being capable of growing at a concentration of 100 nmol/L of bortezomib. Hereinafter, thus prepared cell line was referred to as "KMS-11/Bor".

OPM-2 cells were suspended in an RPMI1640 medium containing 10% fetal calf serum (FCS) at a concentration of $5 \times 10^4$ cells/mL, and incubated at 37˚C in a 5% carbon dioxide incubator. Then, bortezomib was added thereto to adjust the final concentration of 2.5 nmol/L, and the incubation was continued. After confirmation of the survival and satisfactory growth of the cells, bortezomib was added at a concentration of 3.75 nmol/L, and the incubation was continued. Then, after confirmation of the survival and satisfactory growth of the cells, the concentration of bortezomib was increased step by step, and the culture and subculture were repeated to create the bortezomib-resistant OPM-2 cells being capable of growing at a concentration of 15 nmol/L of bortezomib. Hereinafter, thus prepared cell line was referred to as "OPM-2/Bor".

<u>Test Example 2 : Test for the growth inhibition to KMS-11 andKMS-11/Bor</u>

A cell solution (90μL/well) of KMS-11 or KMS-11/Bor suspended in an RPMI1640 medium containing 10% FCS (hereinafter referred to as culture medium) at a concentration of $5 \times 10^4$ cells/mL was inoculated in a 96-well microplate (Nunc), and as for a blank the culture medium (100μL/well each) was added, and the plate was incubated in a 5% carbon dioxide incubator for 5 hours.

The test compound was serially diluted to yield a diluted solution having a concentration of 10-fold of the final

concentration, and the solution (10μL/well each) was added (n = 3 for one concentration). As for a control, the culture medium containing no test compound (10μL/well each) was added. The tests for the control and the blank were performed in n = 8. The incubation was carried out for 72 hours after addition of the test compound, and the number of viable cells was counted with a cell proliferation reagentWST-1 (WST-1 reagent; Roche Diagnostics). The WST-1 reagent (10μL/well) was added to each plate. The plate was incubated in a carbon dioxide incubator for 2 hours, then stirred on a plate mixer, and the absorbance was measured at 450nm and 655nm using a microplate spectrophotometer (Molecular Device; SPECTRAmax 340PC-384). For each well, the differential absorbance was calculated by subtracting the absorbance at 655nm from that at 450nm. Further, the absorbance corresponding to the viable cell number was calculated by subtracting the differential absorbance of the blank from the above differential absorbance.

The followings describe the calculation method for $IC_{50}$. The absorbance corresponding to the viable cell number obtained in the wells untreated with the test compound was regarded as 100%, and the absorbance obtained in the wells treated with the test compound was represented by the relative value (% control). From these values, the $IC_{50}$ of viable cell number (the concentration at which the viable cell number is reduced to 50% of the control) was calculated. Figs.1 to 4 show the % control at the respective concentration of the test compound after treatment with the test compound. Table 3 shows the $IC_{50}$ of each test compound. In addition, values in which $IC_{50}$ of KMS-11/Bor is divided by $IC_{50}$ of KMS-11 ($IC_{50}$ ratio) is shown in Table 3 as the degree of bortezomib resistance in the respective test compounds.

The $IC_{50}$ ratios for bortezomib and MG-132 were 21 and 7.7, respectively, clearly indicating that the growth-inhibitory effect on KMS-11/Bor is decreased. On the other hand, the $IC_{50}$ ratios for 17-AAG and Compound 22 were 0.76 and 0.96, respectively, indicating that decrease of the growth-inhibitory effect on KMS-11/Bor was not recognized.

From the above results, it became apparent that the Hsp90 family protein inhibitors are useful in treatment of a cancer resistant to a protease inhibitor.

Table 3: Growth-inhibitory effect of the test compounds on KMS-11 and KMS-11/Bor

| | $IC_{50}$ (μmol/L) | | $IC_{50}$ ratio |
|---|---|---|---|
| Test compound | KMS-11 | KMS-11/Bor | - |
| MG-132 | 0.26 | 2.0 | 7.7 |
| Bortezomib | 0.0082 | 0.17 | 21 |
| 17-AAG | 0.082 | 0.062 | 0.76 |
| Compound 22 | 0.71 | 0.68 | 0.96 |

Test Example 3: Test for the growth inhibition to OPM-2 and OPM-2/Bor

A cell solution (90μL/well) of OPM-2 or OPM-2/Bor suspended in an RPMI1640 medium containing 10% FCS (hereinafter referred to as culture medium) at a concentration of $5 \times 10^4$ cells/mL was inoculated in a 96-well microplate (Nunc), and as for a blank the culture medium (100μL/well each) was added, and the plate was incubated in a 5% carbon dioxide incubator for 5 hours.

The test compound was serially diluted to yield a diluted solution having a concentration of 10-fold of the final concentration and the solution (10μL/well each) was added (n = 3 for one concentration). As for a control, the culture medium containing no test compound (10μL/well each) was added. The tests for the control and the blank were performed in n = 8. The incubation was carried out for 72 hours after addition of the test compound, and the number of viable cells was counted with a cell proliferation reagent WST-1 (WST-1 reagent; Roche Diagnostics). The WST-1 reagent (10μL/well) was added to each plate. The plate was incubated in a carbon dioxide incubator for 2 hours, then stirred on a plate mixer, and the absorbance was measured at 450nm and 655nm using a microplate spectrophotometer (Molecular Device; SPECTRAmax 340PC-384). For each well, the differential absorbance was calculated by subtracting the absorbance at 655nm from that at 450nm. Further, the absorbance corresponding to the viable cell number was calculated by subtracting the differential absorbance of the blank from the above differential absorbance.

The followings describe the calculation method for $IC_{50}$. The absorbance corresponding to the viable cell number obtained in the wells untreated with the test compound was regarded as 100%, and the absorbance obtained in the wells treated with the test compound was represented by the relative value (% control). From these values, the $IC_{50}$ of viable cell number (the concentration at which the viable cell number is reduced to 50% of the control) was calculated. Figs.5 to 8 show the % control at the respective concentration of the test compound after treatment with the

test compound. Table 4 shows the $IC_{50}$ of each test compound. In addition, values in which $IC_{50}$ ratio for OPM-2/Bor was divided by $IC_{50}$ of OPM-2 ($IC_{50}$ ratio) is shown in Table 4 as the degree of bortezomib resistance in the respective test compounds.

The $IC_{50}$ ratios for bortezomib and MG-132 were 28 and 16, respectively, clearly indicating that the growth-inhibitory effect on OPM-2/Bor is decreased in its sensitivity. On the other hand, the $IC_{50}$ ratios for 17-AAG and Compound 22 were 1.1 and 1.4, respectively, indicating that decrease of the sensitivity to OPM-2/Bor was not recognized.

From the above results, it became apparent that the Hsp90 family protein inhibitors are useful in treatment of a cancer resistant to a protease inhibitor.

Table 4: Growth-inhibitory effect of the test compounds on OPM-2 and OPM-2/Bor

| | $IC_{50}$ (μmol/L) | | $IC_{50}$ ratio |
|---|---|---|---|
| Test compound | OPM-2 | OPM/Bor | - |
| MG-132 | 0.094 | 1.5 | 16 |
| bortezomib | 0.0039 | 0.11 | 28 |
| 17-AAG | 0.31 | 0.35 | 1.1 |
| Compound 22 | 0.34 | 0.48 | 1.4 |

Test Example 4: Anti-tumor effect in a mouse model to which the OPM-2/Bor cells have been implanted

The daybefore the implantation of cancer cells, an anti-asialo GM1 antibody (0.3mg/mouseeach) was administered intraperitoneally to Fox C.B-17/Icr-scidJcl mice (CLEA Japan, Inc.). The OPM-2/Bor cells were grown by incubation in an RPMI1640 medium containing 10% FCS at 37°C in a 5% carbon dioxide incubator, and implanted into the ventral subcutaneous portion at a dose of $1 \times 10^7$ cells/mouse. Eighteen days after implantation, the major axis and minor axis of the tumor growing in the subcutaneous portion were measured with a vernier micrometer, and the tumor volume was calculated according to the following equation.

$$\underset{(mm^3)}{\text{Tumor volume}} = \underset{(mm)}{\text{major axis}} \times \underset{(mm)}{\text{minor axis}} \times \underset{(mm)}{\text{minor axis}} \times \frac{1}{2}$$

At the same time, the body weight of each mouse was measured, and the mice were divided into the following administration groups consisting of 5 mice per group so that the average tumor volume and the average body weight became uniform; and this day was regarded as the test-starting day 0, on which the administration was started.

Compound 22 was dissolved in physiological saline (Otsuka Pharmaceutical Co., Ltd.) at a concentration of 10 or 5 mg/mL, and administered intravenously in the tail vein at a dose of 0.01 mL/g body weight of mouse, respectively, (corresponding to 100 mg/kg and 50 mg/kg) twice a day continuously up to the 4th day from the starting day 0.

Bortezomib was dissolved in physiological saline at a concentration of 0.1 or 0.05 mg/mL, and administered intravenously in the tail vein at a dose of 0.01 mL/g body weight of mouse, respectively, (corresponding to 1 mg/kg and 0.5 mg/kg) once a day on the starting day 0, day 7 and day 10. The dose of bortezomib and the regimen were determined referring to the literature reporting the anti-tumor effect of bortezomib in a mouse model to choose the dose and regimen in which bortezomib would exhibit a remarkable effect [Cancer Research, 62, 4996-5000 (2002)].

A. Negative control group (Control) : No administration of the test compound
B. Bortezomib administered group: 1 mg/kg (administered once a day on the days 0, 3, 7, and 10)
C. Bortezomib administered group: 0.5 mg/kg (administered once a day on the days 0, 3, 7, and 10)
D. Compound 22 administered group: 100 mg/kg (twice a day × 5 days)
E. Compound 22 administered group: 50 mg/kg (twice a day × 5 days)

Following the day 0, the tumor volume was measured twice a week. The anti-tumor effect was determined by calculating the average value of the tumor volume in each group and comparing a change of the tumor volume (V/V0), wherein the tumor volume on the day 0 was defined as V0. Fig. 9 shows V/V0 of each group measured each day.

As shown in Fig. 9, Compound 22 exhibited a strong growth inhibitory effect depending on the dose as well as a tumor reducing effect. On the other hand, bortezomib did not show a remarkable growth inhibitory effect. That is, the Hsp90 family protein inhibitor Compound 22 had a high anti-tumor effect in a mouse model in which the cells irresponsive to bortezomib were implanted.

Table 5 shows the T/C values which were obtained by dividing the V/V0 of each group by the V/V0 of the negative control group on Day 14. The T/C values in the Compound 22-administered group, were 0.092 in D group and 0.24 in E group, indicating that the growth of tumor was significantly inhibited. The T/C values in the bortezomib-administered group were 0.85 in B group and 1.0 in C group, indicating that the effect was equivalent to that of the negative control group.

Table 5: T/C value in each group

| A. Negative Control | B. Test Compound | C. Test Compound | D. Test Compound | E. Test Compound |
|---|---|---|---|---|
| 1.0 | 0.85 | 1.0 | 0.092 | 0.24 |

From the above results, it was revealed that the Hsp90 family protein inhibitors are useful in treatment of a cancer resistant to a protease.

Test Example 5: Comparative test for the expressed amount of Hsp70, Hsp27, Bcl-2 and β-actin in OPM-2 and OPM-2/Bor

A cell solution (2mL/well each) of OPM-2 or OPM-2/Bor suspended in an RPMI1640 medium containing 10% FCS (hereinafter referred to as culture medium) at a concentration of $2 \times 10^5$ cells/mL was inoculated in a 6-well microplate (Corning International), and incubated in a 5% carbon dioxide incubator for 24 hours. The cells were recovered as precipitate, then dissolved in an NP40 cell lysis buffer (Invitrogen) containing 1% protease inhibitor cocktail (Sigma Aldrich) and 1 mmol/L phenylmethylsulfonyl fluoride (PMSF) on ice for 30 minutes, and centrifuged at 15,000G for 10 minutes. The protein concentration in the resulting supernatant was measured, and samples were prepared so that the amount of proteins became the same in the respective lanes, were subjected to separation of the proteins on SDS-PAGE. The separated protein specimens were transferred onto a polyvinylidene fluoride (PVDF) membrane (Millipore), to which was added as primary antibody, anti-Hsp70 antibody (SPA-810, Assay Design), anti-Hsp27 antibody (#2402, Cell Signaling Technology), anti-Bcl-2 antibody (sc-509, Santa Cruz Biotechnology), or anti-β-actin antibody (AC-15, Sigma Aldrich) to react with the proteins on the membrane. Then, as a secondary antibody, horseradish peroxidase-labeled secondary antibody (anti-rabbit Ig antibody or anti-mouse Ig antibody; GE Health Care Bioscience) capable of reacting with the respective primary antibodies was allowed to react with the primary antibodies. The detection was achieved with an ECL reagent (No. 34080; PIERCE Biotechnology), and the bands obtained on the X-ray filmwere examined to compare the amount of Hsp70, Hsp27, Bcl-2 and β-actin proteins expressed in OPM-2 and OPM-2/Bor. Fig. 10 shows the results. According to Fig. 10, it was revealed that there was no difference in the expressed amount of β-actin between the both cell lines, but the expressed amount of Hsp70, Hsp27 and Bcl-2 was increased in OPM-2/Bor in comparison with OPM-2. Thus, it is considered that increase of the expression of these proteins contributes to the bortezomib resistance.

From the above results, it was revealed that the therapeutic agents of the present invention are effective in treatment of a cancer in which the expression of Hsp70, Hsp27 and Bcl-2 is increased. In addition, it was also elucidated that the Hsp90 family protein inhibitors are effective in treatment of a cancer which have a resistance to a protease inhibitor and in which the expression of Hsp70, Hsp27 and Bcl-2 is increased.

Example1

Preparation Example 1(Tablet)

Tablet having the following formulation is prepared in a conventional manner.

| | |
|---|---|
| Compound 1 | 5 mg |
| Lactose | 60 mg |
| Potato Starch | 30 mg |

(continued)

| | |
|---|---|
| Poly(vinyl alcohol) | 2 mg |
| Magnesium stearate | 1 mg |
| Tar pigment | trace amount |

Example 2

Preparation Example 2(Injection)

Injection having the following formulation is prepared in a conventional manner.

| | |
|---|---|
| Compound 17 | 2 mg |
| D-mannitol | 10 mg |
| Aqueous hydrochloric acid solution | proper quantity |
| Aqueous sodium hydroxide solution | proper quantity |
| Distilled water for injection | proper quantity |

Industrial Applicability

The present invention provides a therapeutic agent a for cancer resistant to a protease inhibitor which comprises, as an active ingredient, an Hsp90 family protein inhibitor, and the like.

## Claims

**1.** A therapeutic agent for a cancer resistant to a protease inhibitor which comprises, as an active ingredient, a Heat Shock Protein 90 (Hsp90) family protein inhibitor.

**2.** The therapeutic agent according to Claim 1 wherein the Hsp90 family protein inhibitor is a benzoyl compound represented by formula (I):

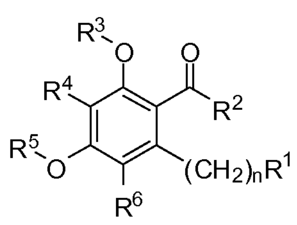

(I)

[wherein n represents an integer of 1 to 5;

$R^1$ represents substituted or unsubstituted lower alkyl, substituted or unsubstituted lower alkoxy, substituted or unsubstituted cycloalkyl, substituted or unsubstituted lower alkoxycarbonyl, substituted or unsubstituted heterocycle-alkyl, substituted or unsubstituted aryl, $CONR^7R^8$ (wherein $R^7$ and $R^8$, which may be the same or different, each represent a hydrogen atom, substituted or unsubstituted lower alkyl, substituted or unsubstituted cycloalkyl, substituted or unsubstituted lower alkanoyl, substituted or unsubstituted aryl, a substituted or unsubstituted heterocyclic group, substituted or unsubstituted aralkyl, substituted or unsubstituted heterocycle-alkyl, or substituted or unsubstituted aroyl, or $R^7$ and $R^8$ form a substituted or unsubstituted heterocyclic group together with the adjacent nitrogen atom thereto), or $NR^9R^{10}$ (wherein $R^9$ and $R^{10}$ have the same meanings as $R^7$ and $R^8$ defined above, respectively);

$R^2$ represents substituted or unsubstituted aryl, or a substituted or unsubstituted aromatic heterocyclic group;

$R^3$ and $R^5$, which may be the same or different, each represent a hydrogen atom, substituted or unsubstituted lower alkyl, substituted or unsubstituted lower alkenyl, substituted or unsubstituted lower alkanoyl, substituted or unsubstituted cycloalkyl, substituted or unsubstituted aralkyl, or substituted or unsubstituted aroyl;

$R^4$ represents a hydrogen atom, hydroxy or halogen; and

$R^6$ represents a hydrogen atom, halogen, cyano, nitro, substituted or unsubstituted lower alkyl, substituted or

unsubstituted lower alkenyl, substituted or unsubstituted lower alkynyl, substituted or unsubstituted lower alkoxy, substituted or unsubstituted cycloalkyl, amino, lower alkylamino, di-lower alkylamino, carboxy, substituted or unsubstituted lower alkoxycarbonyl, substituted or unsubstituted lower alkanoyl, substituted or unsubstituted aryloxy, substituted or unsubstituted aryl, a substituted or unsubstituted heterocyclic group, substituted or unsubstituted aralkyl, or substituted or unsubstituted heterocycle-alkyl] or a pharmaceutically acceptable salt thereof.

**3.** The therapeutic agent according to claim 2 wherein $R^2$ is aryl substituted with 1 to 3 substituents or aryl.

**4.** The therapeutic agent according to claim 2 wherein $R^2$ is phenyl substituted with 1 to 3 substituents or phenyl.

**5.** The therapeutic agent according to claim 2 wherein $R^2$ is phenyl substituted with lower alkoxy and/or heterocycle-alkyloxy.

**6.** The therapeutic agent according to claim 2 wherein $R^2$ is a substituted or unsubstituted aromatic heterocyclic group.

**7.** The therapeutic agent according to any of claims 2 to 6 wherein $R^3$ and $R^5$, which may be the same or different, each are a hydrogen atom, substituted or unsubstituted lower alkyl, substituted or unsubstituted lower alkanoyl, substituted or unsubstituted aroyl, or substituted or unsubstituted lower alkenyl.

**8.** The therapeutic agent according to any of claims 2 to 6 wherein $R^3$, $R^4$ and $R^5$ are hydrogen atoms.

**9.** The therapeutic agent according to any of claims 2 to 8 wherein $R^1$ is $CONR^7R^8$ (wherein $R^7$ and $R^8$ have the same meanings as defined above, respectively).

**10.** The therapeutic agent according to any of claims 2 to 8 wherein $R^1$ is $CONR^{7a}R^{8a}$ (wherein $R^{7a}$ and $R^{8a}$, which may be the same or different, each represent a hydrogen atom, substituted or unsubstituted lower alkyl, or substituted or unsubstituted heterocycle-alkyl).

**11.** The therapeutic agent according to any of claims 2 to 8 wherein $R^1$ is $CONR^{7b}R^{8b}$ (wherein $R^{7b}$ and $R^{8b}$ form a substituted or unsubstituted heterocyclic group together with the adjacent nitrogen atom thereto).

**12.** The therapeutic agent according to any of claims 2 to 8 wherein $R^1$ is $CONR^{7c}R^{8c}$ (wherein $R^{7c}$ and $R^{8c}$, which may be the same or different, each represent 2-hydroxyethyl or 2-methoxyethyl).

**13.** The therapeutic agent according to any of claims 2 to 8 wherein $R^1$ is substituted or unsubstituted lower alkoxy.

**14.** The therapeutic agent according to any of claims 2 to 13 wherein n is 1.

**15.** The therapeutic agent according to any of claims 2 to 14 wherein $R^6$ is a hydrogen atom, lower alkyl, halogen or aryl.

**16.** The therapeutic agent according to any of claims 2 to 14 wherein $R^6$ is lower alkyl.

**17.** The therapeutic agent according to any of claims 2 to 14 wherein $R^6$ is ethyl.

**18.** The therapeutic agent according to claim 1 wherein the Hsp90 familyprotein inhibitor is a benzoyl compound represented by formula (IA) :

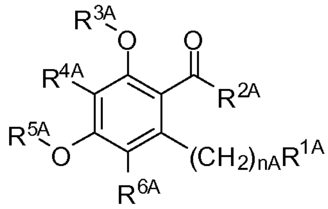

(IA)

[wherein nA represents an integer of 0 to 10;
$R^{1A}$ represents a hydrogen atom, hydroxy, cyano, carboxy, nitro, halogen, substituted or unsubstituted lower alkyl,

substituted or unsubstituted lower alkenyl, substituted or unsubstituted lower alkynyl, substituted or unsubstituted lower alkoxy, substituted or unsubstituted cycloalkyl, substituted or unsubstituted lower alkoxycarbonyl, substituted or unsubstituted lower alkanoyloxy, substituted or unsubstituted heterocycle-alkyl, substituted or unsubstituted aryl, substituted or unsubstituted arylsulfonyl, a substituted or unsubstituted heterocyclic group, $CONR^7R^8$ (wherein $R^7$ and $R^8$ have the same meanings as defined above, respectively), or $NR^9R^{10}$ (wherein $R^9$ and $R^{10}$ have the same meanings as defined above, respectively);

$R^{2A}$ represents substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group;

$R^{3A}$ and $R^{5A}$, which may be the same or different, each represent a hydrogen atom, substituted or unsubstituted lower alkyl, substituted or unsubstituted lower alkenyl, substituted or unsubstituted lower alkanoyl, substituted or unsubstituted cycloalkyl, substituted or unsubstituted aralkyl, or substituted or unsubstituted aroyl; and

$R^{4A}$ and $R^{6A}$, which may be the same or different, each represent a hydrogen atom, hydroxy, halogen, cyano, nitro, substituted or unsubstituted lower alkyl, substituted or unsubstituted lower alkenyl, substituted or unsubstituted lower alkynyl, substituted or unsubstituted lower alkoxy, substituted or unsubstituted cycloalkyl, amino, lower alkylamino, di-lower alkylamino, carboxy, substituted or unsubstituted lower alkoxycarbonyl, substituted or unsubstituted lower alkanoyl, substituted or unsubstituted aryloxy, substituted or unsubstituted aryl, a substituted or unsubstituted heterocyclic group, substituted or unsubstituted aralkyl, or substituted or unsubstituted heterocycle-alkyl] or a pharmaceutically acceptable salt thereof.

**19.** The therapeutic agent according to claim 1 wherein the Hsp90 family protein inhibitor is a benzene derivative represented by formula (II):

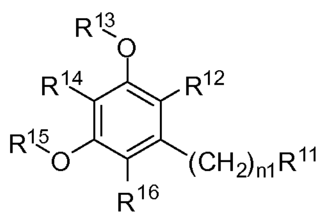

(II)

[wherein $n^1$ represents an integer of 0 to 10;

$R^{11}$ represents a hydrogen atom, hydroxy, cyano, carboxy, nitro, halogen, substituted or unsubstituted lower alkyl, substituted or unsubstituted lower alkenyl, substituted or unsubstituted lower alkynyl, substituted or unsubstituted cycloalkyl, substituted or unsubstituted lower alkoxycarbonyl, substituted or unsubstituted aroyl, substituted or unsubstituted lower alkanoyl, substituted or unsubstituted heterocycle-alkyl, substituted or unsubstituted aryl, substituted or unsubstituted aralkyl, substituted or unsubstituted arylsulfonyl, a substituted or unsubstituted heterocyclic group, $CONR^{17}R^{18}$ (wherein $R^{17}$ and $R^{18}$, which may be the same or different, each represent a hydrogen atom, substituted or unsubstituted lower alkyl, substituted or unsubstituted cycloalkyl, substituted or unsubstituted lower alkanoyl, substituted or unsubstituted aryl, a substituted or unsubstituted heterocyclic group, substituted or unsubstituted aralkyl, substituted or unsubstituted heterocycle-alkyl, or substituted or unsubstituted aroyl, or $R^{17}$ and $R^{18}$ form a substituted or unsubstituted heterocyclic group together with the adjacent nitrogen atom thereto), $NR^{19}R^{20}$ [wherein $R^{19}$ and $R^{20}$, which may be the same or different, each represent a hydrogen atom, substituted or unsubstituted lower alkylsulfonyl, substituted or unsubstituted lower alkyl, substituted or unsubstituted cycloalkyl, substituted or unsubstituted lower alkanoyl, substituted or unsubstituted aryl, a substituted or unsubstituted heterocyclic group, substituted or unsubstituted aralkyl, substituted or unsubstituted heterocycle-alkyl, substituted or unsubstituted aroyl, or $CONR^{21}R^{22}$ (wherein $R^{21}$ and $R^{22}$ have the same meanings as $R^{17}$ and $R^{18}$ defined above, respectively), or $R^{19}$ and $R^{20}$ form a substituted or unsubstituted heterocyclic group together with the adjacent nitrogen atom thereto], or $OR^{23}$ (wherein $R^{23}$ represents substituted or unsubstituted lower alkyl, substituted or unsubstituted lower alkenyl, substituted or unsubstituted lower alkanoyl, substituted or unsubstituted aryl, a substituted or unsubstituted heterocyclic group, substituted or unsubstituted aralkyl, or substituted or unsubstituted heterocycle-alkyl);

$R^{12}$ represents substituted or unsubstituted lower alkyl, substituted or unsubstituted lower alkenyl, substituted or unsubstituted lower alkynyl, substituted or unsubstituted aryl, or a substituted or unsubstituted heterocyclic group;

$R^{13}$ and $R^{15}$, which may be the same or different, each represent a hydrogen atom, substituted or unsubstituted lower alkyl, substituted or unsubstituted lower alkenyl, substituted or unsubstituted lower alkanoyl, substituted or unsubstituted cycloalkyl, substituted or unsubstituted lower alkylsulfonyl, substituted or unsubstituted arylsulfonyl,

carbamoyl, sulfamoyl, substituted or unsubstituted lower alkylaminocarbonyl, substituted or unsubstituted di-lower alkylaminocarbonyl, substituted or unsubstituted lower alkoxycarbonyl, substituted or unsubstituted heterocycle-carbonyl, substituted or unsubstituted aralkyl, or substituted or unsubstituted aroyl; and
$R^{14}$ and $R^{16}$, which may be the same or different, each represent a hydrogen atom, hydroxy, halogen, cyano, nitro, substituted or unsubstituted lower alkyl, substituted or unsubstituted lower alkenyl, substituted or unsubstituted lower alkynyl, substituted or unsubstituted lower alkoxy, substituted or unsubstituted cycloalkyl, amino, lower alkylamino, di-lower alkylamino, carboxy, substituted or unsubstituted lower alkoxycarbonyl, substituted or unsubstituted aryloxy, substituted or unsubstituted aryl, a substituted or unsubstituted heterocyclic group, substituted or unsubstituted lower alkanoyl, substituted or unsubstituted aralkyl, or substituted or unsubstituted heterocycle-alkyl] or a pharmaceutically acceptable salt thereof.

**20.** The therapeutic agent according to claim 19 wherein $R^{11}$ is a hydrogen atom, hydroxy, cyano, carboxy, nitro, halogen, substituted or unsubstituted lower alkyl, substituted or unsubstituted lower alkynyl, substituted or unsubstituted lower alkoxy, substituted or unsubstituted cycloalkyl, substituted or unsubstituted lower alkoxycarbonyl, substituted or unsubstituted lower alkanoyloxy, substituted or unsubstituted heterocycle-alkyl, substituted or unsubstituted aryl, substituted or unsubstituted arylsulfonyl, $CONR^{17}R^{18}$ (wherein, $R^{17}$ and $R^{18}$ have the same meanings as defined above, respectively), or $NR^{19}R^{20}$ (wherein, $R^{19}$ and $R^{20}$ have the same meanings as defined above, respectively).

**21.** The therapeutic agent according to claim 19 wherein $R^{11}$ is substituted or unsubstituted lower alkyl, substituted or unsubstituted lower alkynyl, substituted or unsubstituted lower alkoxy, substituted or unsubstituted cycloalkyl, substituted or unsubstituted lower alkoxycarbonyl, substituted or unsubstituted heterocycle-alkyl, substituted or unsubstituted aryl, $CONR^{17}R^{18}$ (wherein $R^{17}$ and $R^{18}$ have the same meanings as defined above, respectively), or $NR^{19}R^{20}$ (wherein $R^{19}$ and $R^{20}$ have the same meanings as defined above, respectively).

**22.** The therapeutic agent according to any of claims 19 to 21 wherein $R^{12}$ is substituted or unsubstituted aryl, or a substituted or unsubstituted aromatic heterocyclic group.

**23.** The therapeutic agent according to any of claims 19 to 21 wherein $R^{12}$ is substituted or unsubstituted aryl.

**24.** The therapeutic agent according to any of claims 19 to 21 wherein $R^{12}$ is substituted or unsubstituted phenyl.

**25.** The therapeutic agent according to any of claims 19 to 21 wherein $R^{12}$ is substituted or unsubstituted furyl.

**26.** The therapeutic agent according to any of claims 19 to 25 wherein $R^{14}$ is a hydrogen atom, hydroxy or halogen.

**27.** The therapeutic agent according to any of claims 19 to 26 wherein $R^{13}$ and $R^{15}$, which may be the same or different, each are a hydrogen atom, substituted or unsubstituted lower alkyl, substituted or unsubstituted lower alkenyl, substituted or unsubstituted lower alkanoyl, substituted or unsubstituted aroyl, substituted or unsubstituted lower alkylaminocarbonyl, substituted or unsubstituted di-lower alkylaminocarbonyl, substituted or unsubstituted lower alkoxycarbonyl, or substituted or unsubstituted heterocycle-carbonyl.

**28.** The therapeutic agent according to any of claims 19 to 25 wherein $R^{13}$, $R^{14}$ and $R^{15}$ are hydrogen atoms.

**29.** The therapeutic agent according to any of claims 1 to 28 wherein the cancer resistant to a protease inhibitor is cancer derived from hematopoietic tumor, breast cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, head and neck cancer, osteosarcoma, melanoma, or cancer derived from brain tumor resistant to a protease inhibitor.

**30.** The therapeutic agent according to any of claims 1 to 28 wherein the cancer resistant to a protease inhibitor is leukemia, myeloma or lymphoma resistant to a protease inhibitor.

**31.** The therapeutic agent according to any of claims 1 to 28 wherein the cancer resistant to a protease inhibitor is multiple myeloma resistant to a protease inhibitor.

**32.** The therapeutic agent according to any of claims 1 to 28 wherein the cancer resistant to a protease inhibitor is a cancer in which expression of Hsp70, Hsp27 or Bcl-2 is increased.

**33.** The therapeutic agent according to any of claims 1 to 32 wherein the protease inhibitor is an antibody having protease inhibiting activity.

**34.** The therapeutic agent according to any of claims 1 to 32 wherein the protease inhibitor is a low-molecular compound having protease inhibiting activity.

**35.** The therapeutic agent according to any of claims 1 to 34 wherein the protease inhibitor is a matrix metalloprotease inhibitor.

**36.** The therapeutic agent according to any of claims 1 to 34 wherein the protease inhibitor is an inhibitor for urokinase plasminogen activator.

**37.** The therapeutic agent according to any of claims 1 to 34 wherein the protease inhibitor is a cathepsin inhibitor.

**38.** The therapeutic agent according to any of claims 1 to 34 wherein the protease inhibitor is a proteasome inhibitor.

**39.** The therapeutic agent according to any of claims 1 to 34 wherein the protease inhibitor is an aminopeptidase inhibitor.

**40.** The therapeutic agent according to claim 38 wherein the proteasome inhibitor is bortezomib.

**41.** The therapeutic agent according to claim 38 wherein the proteasome inhibitor is MG-132.

**42.** A method for treating a cancer resistant to a protease inhibitor, which comprises a step of adminstering an Hsp90 family protein inhibitor to a patient in need thereof.

**43.** The method according to claim 42 wherein the Hsp90 family protein inhibitor is the benzoyl compound described in any of claims 2 to 17 or a pharmaceutically acceptable salt thereof.

**44.** The method according to claim 42 wherein the Hsp90 family protein inhibitor is the benzoyl compound described in Claim 18 or a pharmaceutically acceptable salt thereof.

**45.** The method according to claim 42 wherein the Hsp90 family protein inhibitor is the benzene derivative described in any of claims 19 to 28 or a pharmaceutically acceptable salt thereof.

**46.** The method according to any of claims 42 to 45 wherein the cancer resistant to a protease inhibitor is the cancer described in any of claims 29 to 32.

**47.** The method according to any of claims 42 to 46 wherein the protease inhibitor is the protease inhibitor described in any of claims 33 to 41.

**48.** Use of an Hsp90 family protein inhibitor for the manufacture of a therapeutic agent for a cancer resistant to a protease inhibitor.

**49.** The use according to claim 48 wherein the Hsp90 family protein inhibitor is the benzoyl compound described in any of claims 2 to 17 or a pharmaceutically acceptable salt thereof.

**50.** The use according to claim 48 wherein the Hsp90 family protein inhibitor is the benzoyl compound described in claim 18 or a pharmaceutically acceptable salt thereof.

**51.** The use according to claim 48 wherein the Hsp90 family protein inhibitor is the benzene derivative described in any of claims 19 to 28 or a pharmaceutically acceptable salt thereof.

**52.** The use according to any of claims 48 to 51 wherein the cancer resistant to a protease inhibitor is the cancer described in any of claims 29 to 32.

**53.** The use according to any of claims 48 to 52 wherein the protease inhibitor is the protease inhibitor described in any of claims 33 to 41.

**54.** A therapeutic agent for a cancer in which expression of Hsp70, Hsp27 or Bcl-2 is increased, which comprises, as an active ingredient, the benzoyl compound or the benzene derivative described in any of claims 2 to 28 or a pharmaceutically acceptable salt thereof as the Hsp90 family protein inhibitor.

**55.** A method for treating a cancer in which expression of Hsp70, Hsp27 or Bcl-2 is increased, which comprises, a step of administering the Hsp90 family protein inhibitor comprising, as an active ingredient, the benzoyl compound or the benzene derivative described in any of claims 2 to 28 or a pharmaceutically acceptable salt thereof to a patient in need thereof.

**56.** Use of an Hsp90 family protein inhibitor which comprises, as an active ingredient, the benzoyl compound or the benzene derivative described in any of claims 2 to 28 or a pharmaceutically acceptable salt thereof for the manufacture of a therapeutic agent for a cancer in which expression of Hsp70, Hsp27 or Bcl-2 is increased.

[Fig. 1]

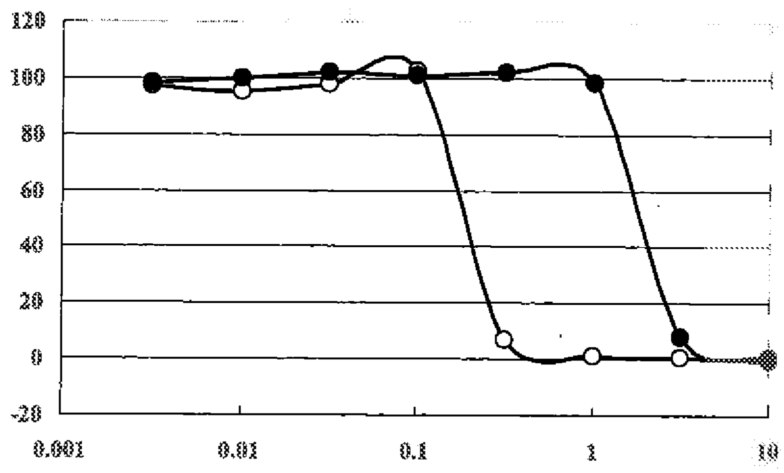
120
100
80
60
40
20
0
-20
0.001
0.01
0.1
1
10

[Fig. 2]

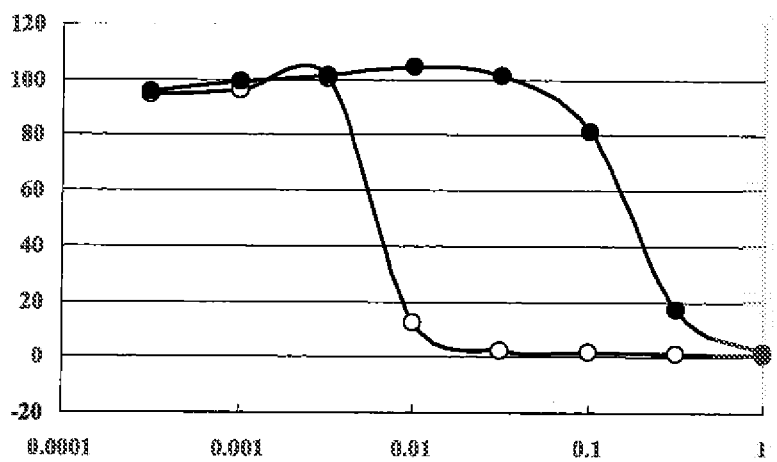
120
100
80
60
40
20
0
-20
0.0001
0.001
0.01
0.1
1

[Fig. 3]

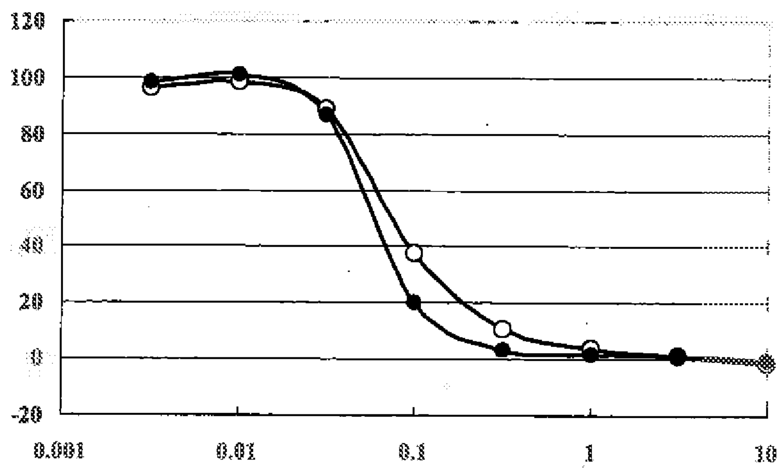
120
100
80
60
40
20
0
-20
0.001
0.01
0.1
1
10

[Fig. 4]

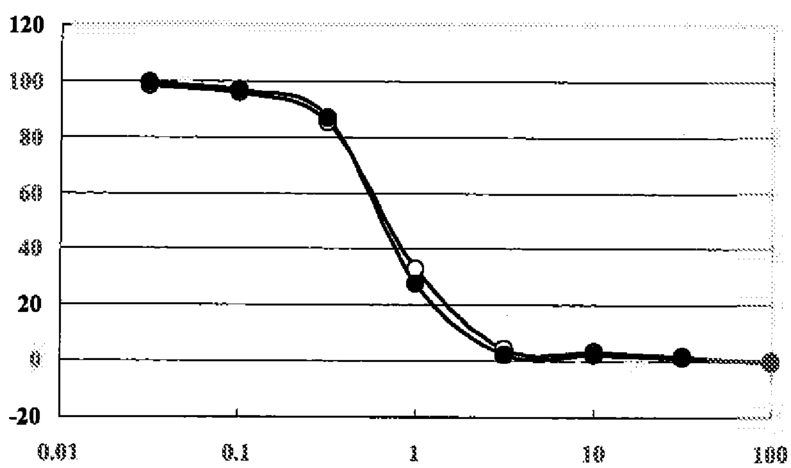
120
100
80
60
40
20
0
-20
0.01
0.1
1
10
100

[Fig. 5]

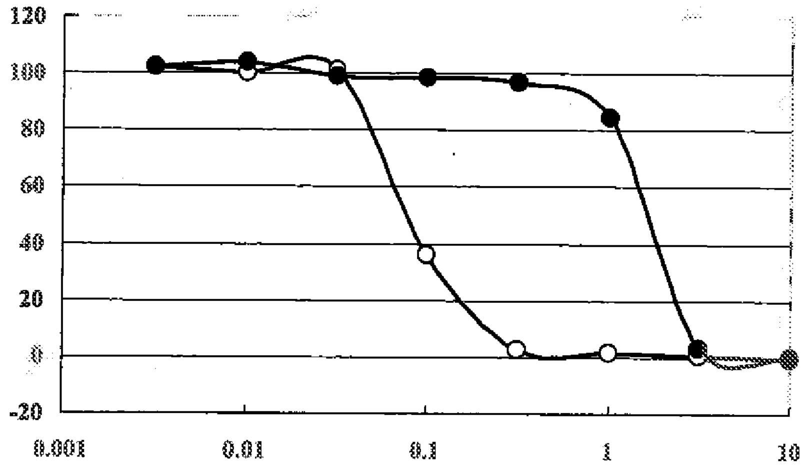
120
100
80
60
40
20
0
-20
0.001
0.01
0.1
1
10

[Fig. 6]

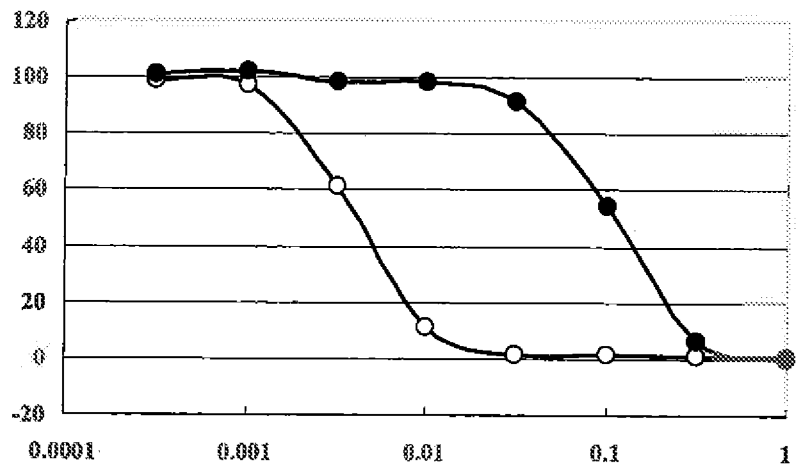
120
100
80
60
40
20
0
-20
0.0001
0.001
0.01
0.1
1

[Fig. 7]

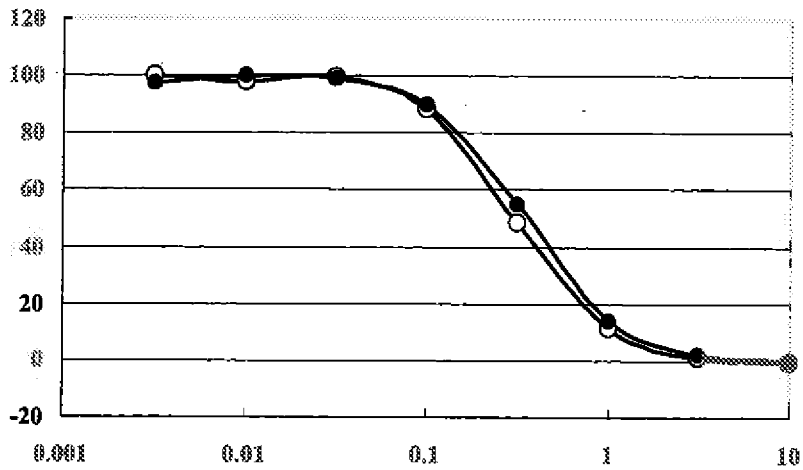
120
100
80
60
40
20
0
-20
0.001
0.01
0.1
1
10

[Fig. 8]

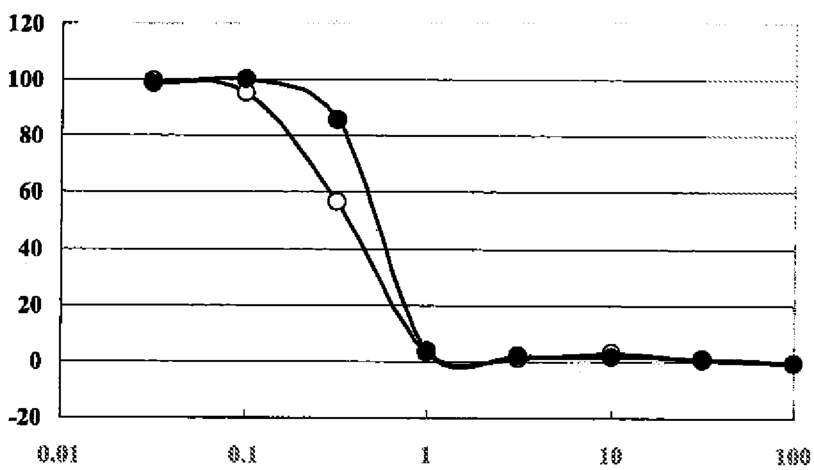
120
100
80
60
40
20
0
-20
0.01
0.1
1
10
100

[Fig. 9]

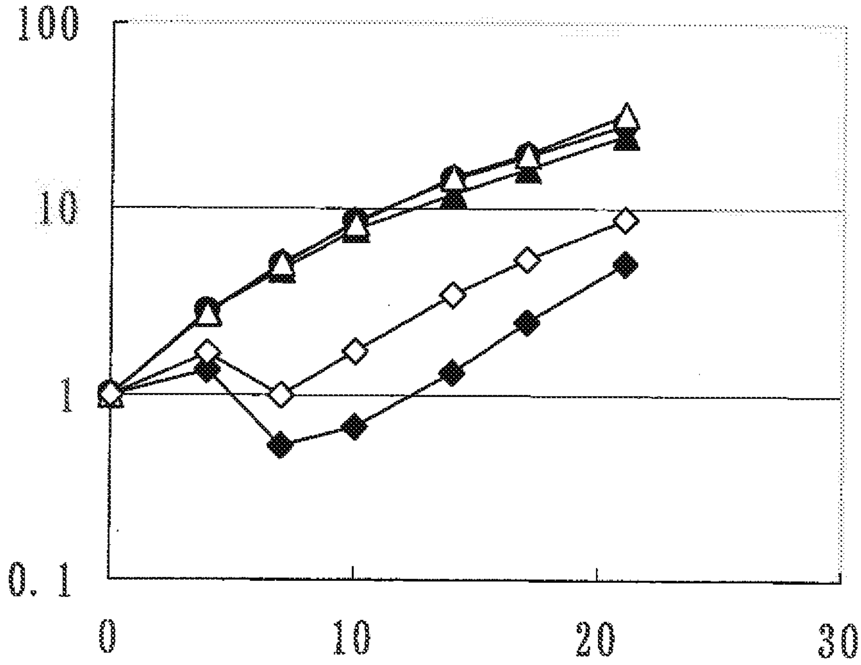

[Fig. 10]

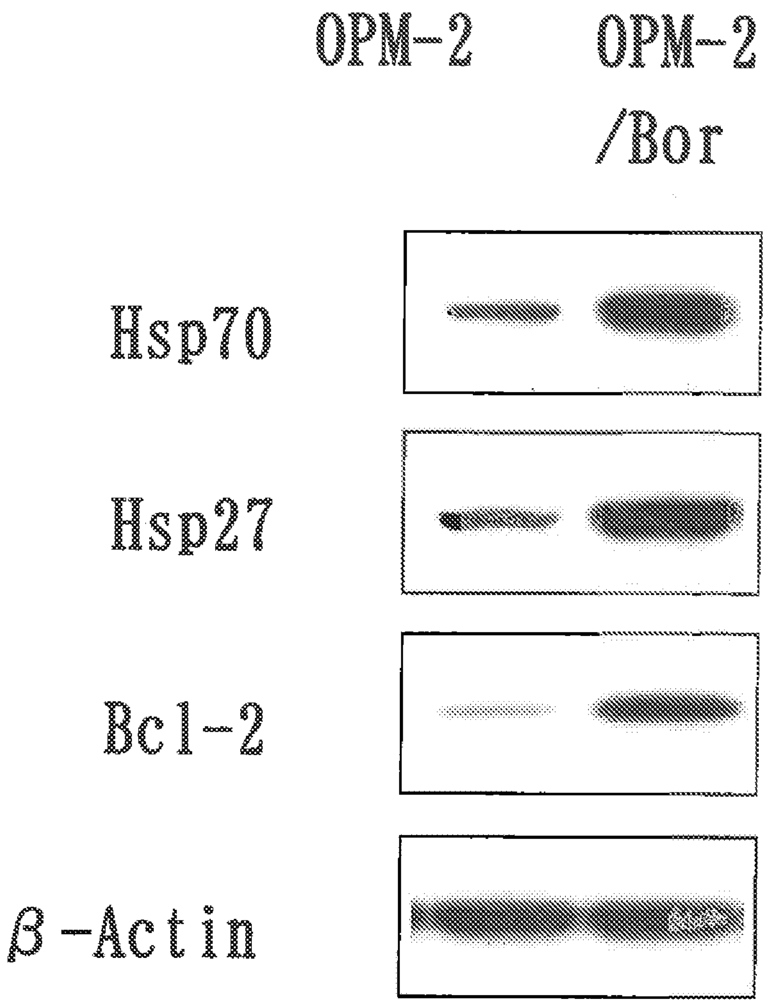

**INTERNATIONAL SEARCH REPORT**

International application No.
PCT/JP2008/064887

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
A61K45/00, A61K31/047, A61K31/12, A61K31/165, A61K31/216, A61K31/4402, A61K31/4406, A61K31/445, A61K31/472, A61K31/495, A61K31/5375, A61P35/00, A61P35/02, A61P43/00, C07D213/30, C07D213/40, C07D217/06, C07D295/08,

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
BIOSIS(STN), CAplus(STN), EMBASE(STN), MEDLINE(STN), JSTPlus(JDreamII), JMEDPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X<br><br>Y | Mitsiades C.S. et al., Antimyeloma activity of heat shock protein-90 inhibition, Blood, 2006, Vol.107, No.3, pages 1029 to 1100, ISSN: 0006-4971 | 1,38,40,48, 52,53<br>2-37,39,41, 49-51,54,56 |
| Y | WO 2005/000778 A1 (Kyowa Hakko Kogyo Co., Ltd.), 06 January, 2005 (06.01.05), Full text; particularly, compound 125; test example 1 & US 2007/0032532 A1 & EP 1642880 A1 | 2-37,39,41, 49-51,54,56 |
| Y | WO 2005/063222 A1 (Kyowa Hakko Kogyo Co., Ltd.), 14 July, 2005 (14.07.05), Full text; particularly, test example 1 & US 2007/0155813 A1 & EP 1704856 A1 | 2-37,39,41, 49-51,54,56 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>24 September, 2008 (24.09.08) | Date of mailing of the international search report<br>07 October, 2008 (07.10.08) |
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

International application No.
PCT/JP2008/064887

C (Continuation). DOCUMENTS CONSIDERED TO BE RELEVANT

| Category* | Citation of document, with indication, where appropriate, of the relevant passages | Relevant to claim No. |
|---|---|---|
| Y | Shringarpure R. et al., Gene expression analysis of B-lymphoma cells resistant and sensitive to bortezomib, British Journal of Haematology, 2006, Vol.134, No.2, pages 145 to 156, ISSN: 0007-1048 | 32-37,39,41, 54,56 |
| Y | Mitsiades N. et al., Molecular sequelae of proteasome inhibition in human multiple myeloma cells, Proc. Natl. Acad. Sci. U.S.A., 2002, Vol.99, No.22, pages 14374 to 14379 | 32-37,39,41, 54,56 |

Form PCT/ISA/210 (continuation of second sheet) (April 2007)

**INTERNATIONAL SEARCH REPORT**

International application No.
PCT/JP2008/064887

Continuation of A. CLASSIFICATION OF SUBJECT MATTER
(International Patent Classification (IPC))

*A61K45/00*(2006.01)i, *A61K31/047*(2006.01)i, *A61K31/12*(2006.01)i, *A61K31/165*(2006.01)i, *A61K31/216*(2006.01)i, *A61K31/4402*(2006.01)i, *A61K31/4406*(2006.01)i, *A61K31/445*(2006.01)i, *A61K31/472*(2006.01)i, *A61K31/495*(2006.01)i, *A61K31/5375*(2006.01)i, *A61P35/00*(2006.01)i, *A61P35/02*(2006.01)i, *A61P43/00*(2006.01)i, *C07D213/30*(2006.01)i, *C07D213/40*(2006.01)i, *C07D217/06*(2006.01)i, *C07D295/08*(2006.01)i, *C07D295/16*(2006.01)i

(According to International Patent Classification (IPC) or to both national classification and IPC)

Continuation of B. FIELDS SEARCHED
Minimum documentation searched (International Patent Classification (IPC))

C07D295/16

Minimum documentation searched (classification system followed by classification symbols)

**INTERNATIONAL SEARCH REPORT**

International application No.
PCT/JP2008/064887

**Box No. II Observations where certain claims were found unsearchable (Continuation of item 2 of first sheet)**

This international search report has not been established in respect of certain claims under Article 17(2)(a) for the following reasons:

1. [x] Claims Nos.: 42-47, 55
because they relate to subject matter not required to be searched by this Authority, namely:
Claims 42 to 47 and 55 involve a method for treatment of a human body by therapy and thus relate to a subject matter which this International Searching Authority is not required, under the provisions of PCT Rule 39.1(iv), to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee.

[ ] The additional search fees were accompanied by the applicant's protest but the applicable protest fee was not paid within the time limit specified in the invitation.

Form PCT/ISA/210 (continuation of first sheet (2)) (April 2007)

INTERNATIONAL SEARCH REPORT

International application No.
PCT/JP2008/064887

<With respect to subject matters for search>

Claims 1, 29-39, 48, 52, 53, and 56 pertain to a therapeutic agent for cancers having resistance to substances defined by the desired property, such as a "protease inhibitor," "antibody having protease inhibitory activity," "low-molecular compound having protease inhibitory activity," "matrix metalloprotease inhibitor," "urokinase plasminogen activator inhibitor," "cathepsin inhibitor," "proteasome inhibitor," and "aminopeptidase inhibitor," the agent containing, as an active ingredient, a substance defined by the desired property, i.e., a "heat shock protein 90 (Hsp90) family protein inhibitor."

Claims 1, 29-39, 48, 52, 53, and 56 involve all substances having such property. However, the substances which are disclosed in the meaning of Article 5 of the PCT are limited to an extremely small part of the substances claimed. Those claims are hence considered to lack a support by the description in the meaning of Article 6 of the PCT.

Even when technical common sense at the time of the filing of this application is taken into account, the terms "heat shock protein 90 (Hsp90) family protein inhibitor," "protease inhibitor," "antibody having protease inhibitory activity," "low-molecular compound having protease inhibitory activity," "matrix metalloprotease inhibitor," "urokinase plasminogen activator inhibitor," "cathepsin inhibitor," "proteasome inhibitor," and "aminopeptidase inhibitor" cannot be used to specify the scope of the compounds having such property. Consequently, claims 1, 29-39, 48, 52, 53, and 56 do not comply also with the requirement of clearness as provided for in Article 6 of the PCT.

Therefore, a search was made for the relationship between the inhibition of a heat shock protein 90 (Hsp90) family protein and the treatment of a cancer having resistance to protease inhibitors, and for the therapeutic agent which is for use in the treatment of cancers having resistance to protease inhibitors described in the description and which contains as an active ingredient the substance specified in claims 2-28 and described in the description.

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005000778 A
- WO 2005063222 A
- WO 2006088193 A
- WO 2007025718 A
- US 20050245435 A
- WO 2007017284 A
- WO 2005105826 A


### Non-patent literature cited in the description


- *Current Medicinal Chemistry,* 2007, vol. 14, 223-232
- *New England Journal of Medicine,* 2001, vol. 344, 1084-1086
- *New England Journal of Medicine,* 2001, vol. 344, 1031-1037
- *Blood,* 2002, vol. 99, 3472-3475
- *Leukemia,* 2003, vol. 17, 829-838
- *Blood,* 2000, vol. 96, 2284-2291
- *Blood,* 2002, vol. 100, 3041-3044
- *Science,* 1987, vol. 235, 177-182
- *Int. J. Cancer,* vol. 49, 650-655
- *Journal of Clinical Oncology,* 1999, vol. 17, 2639-2648
- *New England Journal of Medicine,* 2001, vol. 344, 783-792
- *Immunology Letters,* 2006, vol. 104, 146-155
- *New England Journal of Medicine,* 2005, vol. 352, 786-792
- *Clinical Cancer Research,* 2007, vol. 12, 6494-6501
- *Cancer Research,* 2005, vol. 65, 6401-6408
- *Journal of Clinical Oncology,* 2003, vol. 21, 4342-4349
- *Journal of Clinical Oncology,* 2006, vol. 24, 4764-4774
- *Clinical Cancer Research,* 2003, vol. 12, 2622-2627
- *Cancer Research,* 2006, vol. 66, 9153-9161
- *New England Journal of Medicine,* 2003, vol. 348, 2609-2617
- *Blood,* 2005, vol. 106, 361
- *Blood,* 2006, vol. 108, 3579
- *Blood,* 2006, vol. 108, 406
- *Blood,* 2003, vol. 102 (1), 269-275
- *Cancer Research,* 2005, vol. 65 (22), 10536-10544
- *Cancer Research,* 2006, vol. 66 (22), 10967-10975
- *Nature Chemical Biology,* 2007, vol. 3, 498-507
- *Expert Opinion on Biological Therapy,* 2002, vol. 2, 3-24
- *Nature Reviews Cancer,* 2006, vol. 6, 227
- *Expert Opinion on Biological Therapy,* 2006, vol. 6, 257
- *Neoplasma,* 2005, vol. 52, 185
- *AACR Annual Meeting,* 2005, 2910
- *Biochemical Journal,* 2004, vol. 381, 511
- *European Journal of Cancer,* 2006, vol. 42, 1623
- *British Journal of Cancer,* 2006, vol. 95, 961
- *Biochemical Pharmacology,* 2004, vol. 67, 227
- *Angiogenesis,* 2004, vol. 7, 91
- *Clinical Cancer Research,* 2006, vol. 12, 2583
- *Proceedings of the American Society for Clinical Oncology,* 2006, vol. 25, 3053
- *Cancer Research,* 2002, vol. 62, 4996-5000